# EUROPEAN PATENT APPLICATION

(11) **EP 1 671 535 A1**
(43) Date of publication of application: **21.06.2006**
(21) Application number: 05027637.7
(22) Date of filing: 16.12.2005
(51) Int. Cl.: A01H 3/00, C12N 15/29, C07K 14/415, C07K 16/16, C12P 17/02, C12N 5/14, A61K 8/365

(54) **Methods for altering levels of phenolic compounds in plant cells**

(30) Priority: 16.12.2004 EP 04029898
(71) Applicant: Stiftung Caesar Center of Advanced European Studies and Research, 53175 Bonn (DE)
(72) Inventor: Cerboncini, Claudio, 53111 Bonn (DE); Schnabel, Heide, 53343 Wachtberg Villip (DE); Theisen, Ralf, 52457 Aldenhoven (DE)
(74) Representative: Müller Fottner Steinecke

(57) **Abstract**

The present invention provides a plant derived extract comprising inhibitory activity against HIV integrase, wherein the amount of inhibitory activity in an extract can be increased by stressing the plant prior to forming an extract. Furthermore, methods and materials for altering levels in plants of one or more phenolic compounds that are intermediates or final products of the plant phenylpropanoid pathway are described. In addition, methods for the isolation of enzymes and their encoding genes, which are involved in the biosynthesis of selected phenolic compounds such as depsides are provided as well as transgenic organisms transformed therewith.

SEQUENCE LISTING

<110> Stiftung caesar

<120> Means and methods for altering levels of phenolic compounds in plant
      cells and transgenic plants

<130> CA18A01/P-EP/EP

<150> EP 04 029 898.6
<151> 2004-12-16

<160> 6

<170> PatentIn version 3.3

<210> 1
<211> 199
<212> DNA
<213> Helianthus maximiliani

<220>
<221> cDNA
<222> (1)..(199)
<223> partial cDNA of differentially expressed mRNA

<400> 1
gcatcaagat agcatagacc ggtgctcatg atgaaatgag agatgatggg tcttcatttt      60

tggggaatga aacttgatgg tcatgattca tgatgatgag aaatattggc acgaaggatt     120

ggatgatgat tatgtgtcag atcatgataa cgaagtgata gatagatggg atagaggaca     180

attgaatcta ttccgtacc                                                  199

<210> 2
<211> 33
<212> DNA
<213> artificial sequence

<220>
<223>  oligonucleotide

<400>  2
cgggcctctt cgctattttt tttttttttt tta                                    33

<210>  3
<211>  33
<212>  DNA
<213>  artificial sequence

<220>
<223>  oligonucleotide

<400>  3
cgggcctctt cgctattttt tttttttttt ttc                                    33

<210>  4
<211>  33
<212>  DNA
<213>  artificial sequence

<220>
<223>  oligonucleotide

<400>  4
cgggcctctt cgctattttt tttttttttt ttg                                    33

<210>  5
<211>  15
<212>  DNA
<213>  artificial sequence

<220>
<223>  oligonucleotide

<400>  5
cgggcctctt cgcta                                                        15

<210> 6
<211> 15
<212> DNA
<213> artificial sequence

<220>
<223> oligonucleotide

<220>
<221> oligonucleotide
<222> (1)..(15)

<400> 6
gcccggagaa gcgat 15

## Description

### Field of the invention

The present invention generally relates to methods and materials for altering levels in plants of one or more phenolic compounds that are intermediates or final products of the plant phenylpropanoid pathway. The present invention further concerns the provision of enzymes and their encoding genes, which are involved in the biosynthesis of selected phenolic compounds such as depsides.

### Background of the invention

Plants provide an almost endless variety of chemical compounds derived from primary or secondary metabolism. Many plant secondary metabolites are desirable. For example, some plant secondary metabolites provide protection against pathogens or adverse environmental conditions, and thus have substantial agronomic importance. In addition, a number of plant secondary metabolites serve as nutraceutical components of our diet. Furthermore, certain plant secondary metabolites have diverse medical applications, particularly in the pharmaceutical industry; see Heilmann and Bauer (1999) Functions of Plant Secondary Metabolites and their Exploitation in Biotechnology. M. Wink. Boca Raton, CRC Press LLC. 3, 274-310.

Plant secondary metabolites can be grouped into several major classes including the phenolics, alkaloids, and isoprenoids. The amino acids phenylalanine and tyrosine serve as precursors for phenolic compounds that are intermediates or final products of a branch of the phenylpropanoid pathway. A schematic representation of the phenylpropanoid pathway which leads from phenylalanine through several branches to the hydroxy cinnamates, lignins, and the flavonoids is shown in figure 1 of US patent application No. 2003/150011, the disclosure content of which is specifically incorporated herein by reference. The phenylpropanoids, and their derivatives, and the flavonoids, and their derivatives, are examples of intermediates and final products of the phenylpropanoid pathway respectively.

The first committed step in the phenylpropanoid pathway is catalyzed by phenylalanine ammonia lyase (PAL), which converts phenylalanine to cinnamic acid (or tyrosine to [rho]-coumaric acid in some monocots). Transcriptional activation of genes encoding enzymes involved in phenylpropanoid metabolism, such as PAL, 4-coumarate CoA ligase (4CL), and cinnamyl alcohol dehydrogenase (CAD), represents a key step in the regulation of the phenylpropanoid pathway.

In view of the important role of phenolic compounds that are intermediates and final products of the plant phenylpropanoid pathway, it is desirable to have means and methods which are capable of regulating the levels of these secondary metabolites in plants.

It is an object of the present invention to provide an economic, simple method for increasing the content of phenolic compounds in plants and to improve their health-promoting properties as well as to provide methods for the identification and cloning of genes involved in the biosynthesis of these compounds. The latter will allow the recombinant production of enzymes which can be used for the biosynthesis of economically, in particular medical important compounds.

The solution to said technical problem is achieved by providing the embodiments characterized in the claims, and described further below.

### Summary of the invention

The present invention provides methods for altering levels in plants of one or more phenolic compounds that are intermediates or final products of the plant phenylpropanoid pathway. One method comprises increasing the content of depsides in a plant, or corresponding tissue or cell thereof, said method comprising treating said plant, tissue or cell with a biotic stimulus such as the plant pathogen *Sclerotinia.* Levels of phenolic compounds in the treated plant cells are assayed using standard techniques such as high performance liquid chromatography (HPLC).

Furthermore, the present invention relates to the use of a plant, tissue or cell as treated in the method of the present invention, with an increased content of depsides, or of products prepared with these plants, tissue or cells such as juice, teas, extracts, fermentation products, and fermentation residues for the preparation of a curative composition, health-promoting composition or tonic, or for a cosmetic. In particular, pharmaceutical compositions for the treatment of viral diseases, advantageously HIV/AIDS are provided.

The present invention also concerns an extract, juice, or press cake with an increased content of depsides, obtainable from a plant, tissue or cell as treated in accordance with a method of the present invention.

It is also an object of the present invention to provide enzymes and nucleic acid molecules correlating with or involved in the biosynthesis or accumulation of depsides in a plant.

It is another object of the present invention to provide a vector comprising a nucleic acid molecule encoding an enzyme involved in the biosynthesis or accumulation of depsides and host cells comprising such a vector, preferably wherein said host cell is a plant cell.

According to another aspect, the invention relates to a method of producing an enzyme involved in the biosynthesis or accumulation of depsides comprising
(a) culturing the mentioned host cell under conditions allowing expression of the nucleic acid molecule; and
(b) isolating the enzyme from the cell or cell culture.

Furthermore, polypeptides encoded by the nucleic acid molecules and/or obtainable by said method are subject of the present invention as well as antibodies recognizing specifically the polypeptide so obtained.

In yet another embodiment of the invention a method of producing a transgenic organism, preferably a plant is provided, comprising introducing into a cell a nucleic acid molecule or a vector as mentioned before. Transgenic cells and organisms obtainable by the described method, preferably wherein the presence of said nucleic acid molecule leads to a change in the content of a depside compared to the wild type organism are also encompassed in the scope of the present invention.

The present invention also relates to a process for the production of depsides comprising
(a) subjecting a substrate comprising phenolic carboxylic acids such as phenylpropanic acids like cinnamic acid, ferulic acid, p-coumaric acid, caffeic acid, 5-hydroxyferulic acid, and/or sinapic acid to a host cell of the present invention or a culture medium thereof, a polypeptide, i.e. enzyme of the present invention or to an extract of a plant that has been modified in accordance with the present invention or fruits thereof; and
(b) isolating the depsides so produced.

The present invention also provides a pharmaceutical composition comprising a pharmaceutically acceptable carrier and a pharmaceutically effective amount of at least one of phenolic compounds such as depsides as defined herein. The pharmaceutical compositions may be used to inhibit integrase, including HIV integrase, thus providing protection against HIV infection.

The expression "pharmaceutically effective amount" is to be understood herein as referring to an amount effective in treating HIV infection in a patient. The term prophylactically effective amount refers to an amount effective in preventing HIV infection in a patient. As used herein, the term patient refers to a mammal, including a human. The expressions "pharmaceutically acceptable carrier" (or adjuvant) and "physiologically acceptable vehicle" are to be understood as referring to a non-toxic carrier or adjuvant that may be administered to a patient, together with a compound of this invention, and which does not destroy the pharmacological activity thereof.

In addition, certain of the compounds that fall within the described bounds of the present invention may form succinic acid and esters and salts with organic and inorganic bases, and it is specifically contemplated that all such salts, in particular pharmaceutically acceptable salts, are included within the present invention

Thus, the compounds obtained by the methods of the present invention are useful as therapeutic and prophylactic agents to treat or prevent infection by HIV-1 and related viruses, which may result in asymptomatic HIV-1 infection, AIDS-related complex (ARC), acquired immunodeficiency syndrome (AIDS), AIDS-related dementia, or similar diseases of the immune system.

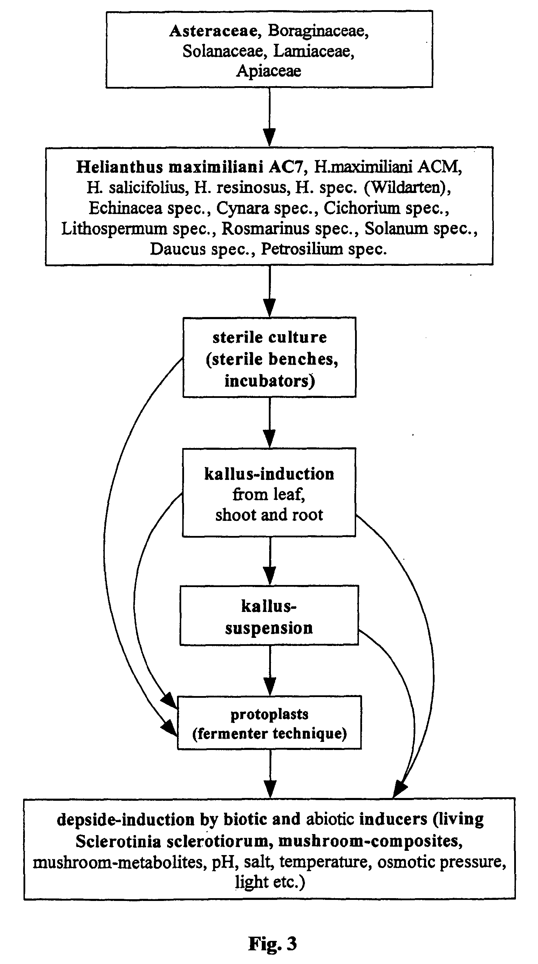

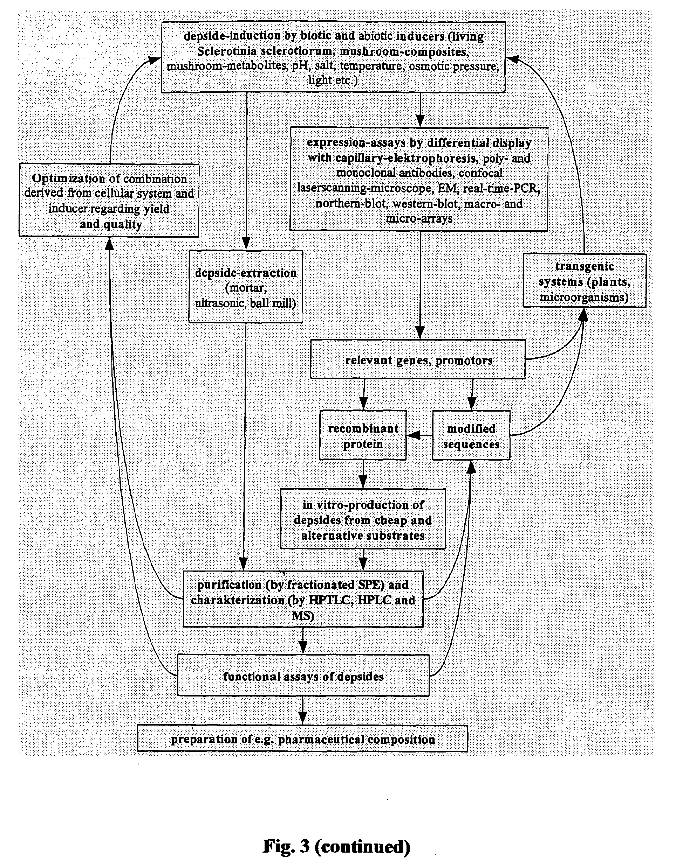

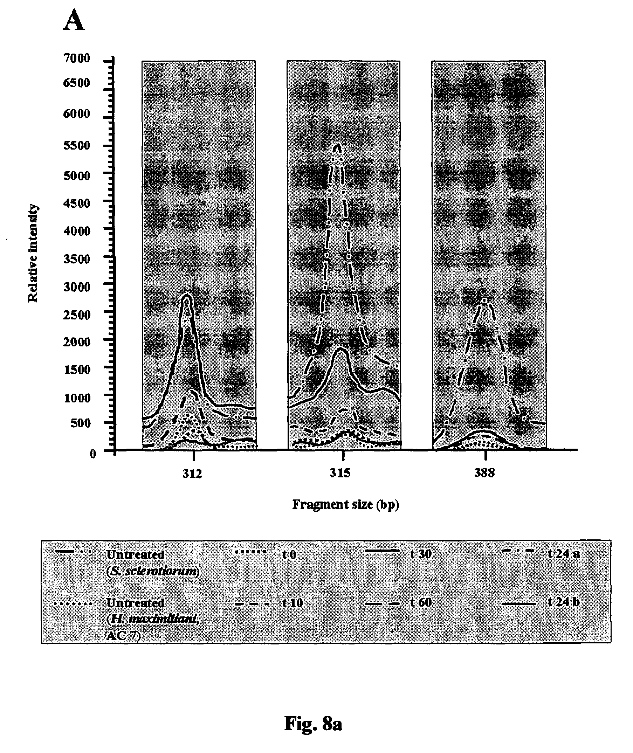

Further embodiments of the present invention are described below and can be inferred from the flow chart shown in Figure 3 and Figure 7.

### Definitions

The terms "nucleic acid", "nucleic acid molecule", "polynucleotide", "polynucleotide sequence" and "nucleic acid sequence" refer to single-stranded or double-stranded deoxyribonucleotide or ribonucleotide polymers, or chimeras thereof. As used herein, the term can additionally or alternatively include analogs of naturally occurring nucleotides having the essential nature of natural nucleotides in that they hybridize to single-stranded or double-stranded nucleic acids in a manner similar to naturally occurring nucleotides (e.g., peptide nucleic acids). Unless otherwise indicated, a particular nucleic acid sequence of this invention optionally encompasses complementary sequences, in addition to the sequence explicitly indicated. The term "gene" is used to refer to, e.g., a cDNA and an mRNA encoded by the genomic sequence, as well as to that genomic sequence.

The term "homologous" refers to nucleic acid sequences that are derived from a common ancestral gene through natural or artificial processes (e.g., are members of the same gene family), and thus, typically, share sequence similarity.

Typically, homologous nucleic acids have sufficient sequence identity that one of the sequences or its complement is able to selectively hybridize to the other under stringent hybridization conditions. The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences have about at least 70% sequence identity, preferably at least 80% sequence identity, more preferably at least 90% sequence identity, and most preferably 95%, 97%, 99%, or 100% sequence identity with each other. A nucleic acid that exhibits at least some degree of homology to a reference nucleic acid can be unique or identical to the reference nucleic acid or its complementary sequence.

Two single-stranded nucleic acids "hybridize" when they form a double-stranded duplex. The region of double-strandedness can include the full-length of one or both of the single-stranded nucleic acids, or all of one single stranded nucleic acid and a subsequence of the other single-stranded nucleic acid, or the region of double-strandedness can include a subsequence of each nucleic acid. An overview of the hybridization of nucleic acids is found in Tijssen (1993) Laboratory Techniques in Biochemistry and Molecular Biology-Hybridization with Nucleic Acid Probes, Part I, Chapter 2 "Overview of Principles of Hybridization and the Strategy of Nucleic Acid Probe Assays", Elsevier, New York.

General texts which discuss considerations relevant to nucleic acid hybridization, the selection of probes, and buffer and incubation conditions, and the like, as well as numerous other topics of interest in the context of the present invention (e.g., cloning of nucleic acids which correlate with the production of phenolic compounds, in particular depsides, sequencing of cloned cDNAs, the use of promoters, vectors, etc.) can be found in Berger and Kimmel (1987) Guide to Molecular Cloning Techniques, Methods in Enzymology vol. 152, Academic Press, Inc., San Diego ("Berger"); Sambrook et al., (2001) Molecular Cloning-A Laboratory Manual, 3rd ed. vols. 1-3, Cold Spring Harbor Laboratory, Cold Spring Harbor ("Sambrook"); and Ausubel et al., (eds) (supplemented through 2001) Current Protocols in Molecular Biology, John Wiley and Sons, Inc., ("Ausubel"). For example, some techniques for detecting genetic markers utilize hybridization of a probe nucleic acid to nucleic acids corresponding to the genetic marker.

The term "isolated" refers to material, such as a nucleic acid or a protein, which is substantially free from components that normally accompany or interact with it in its naturally occurring environment. The isolated material optionally comprises material not found with the material in its natural environment, e.g., a cell. In addition, if the material is in its natural environment, such as a cell, the material has been placed at a location in the cell (e.g., genome or subcellular organelle) not native to a material found in that environment. For example, a naturally occurring nucleic acid (e.g., a promoter) is considered to be isolated if it is introduced by non-naturally occurring means to a locus of the genome not native to that nucleic acid. Nucleic acids which are "isolated" as defined herein, are also referred to as "heterologous" nucleic acid molecules.

The term "antisense" as used herein refers to a single-stranded nucleic acid, typically RNA, having a complementary base sequence to the base sequence of a messenger RNA (mRNA). The term "complementary" as used herein refers to a nucleotide sequence that is related to another nucleotide sequence by the Watson-Crick base-pairing rules, i.e., the sequence A-T-G-C in a DNA strand is complementary to the sequence T-A-C-G in a second DNA strand and to the sequence U-A-C-G in an RNA strand.

Double-stranded RNA (dsRNA) as used herein refers to polyribonucleotide structure formed by either a single self-complementary RNA strand or by at least two complementary RNA strands. The degree of complementary does not necessarily need to be 100 percent. Rather, it must be sufficient to allow the formation of a double-stranded structure under the conditions employed.

Gene expression or expression as used herein refers to the presence of an RNA transcribed from a gene or a protein translated from an RNA transcribed from the gene within a cell, tissue, or organism. More specifically, gene expression can be evaluated with respect to RNA expression or protein expression. The term "gene expression" is also used to refer to the process by which RNA is transcribed from a gene or by which RNA transcribed from a gene is translated.

The term "sense" as used herein, refers to a base sequence as present in a messenger RNA (mRNA).

The term "vector" as used herein, refers to a nucleic acid molecule capable of mediating introduction of another nucleic acid to which it has been linked into a cell. One type of preferred vector is an episome, i.e., a nucleic acid capable of extrachromosomal replication. Preferred vectors are those capable of extra-chromosomal replication and/or expression of nucleic acids to which they are linked in a host cell. Vectors capable of directing the expression of inserted DNA sequences are referred to herein as "expression vectors" and may include plasmids or viruses, in particular baculoviruses. However, the invention is intended to include such other forms of expression vector which serve equivalent functions and which become known in the art subsequently hereto.

The present invention provides sense, antisense, and dsRNAi expression constructs which can be used to prepare transgenic plant cells, plant parts, and plants having elevated or depressed levels of one or more phenolic compounds that are intermediates or final products of the phenylpropanoid pathway. One preferred example of phenolic compounds that are believed to be the final product of one branch of the phenylpropanoid pathway are depsides. It is also believed that cinnamic acid, ferulic acid, p-coumaric acid, caffeic acid, 5-hydroxyferulic acid, and/or sinapic acid are precursor required for depside formation. The depsides dicaffeoylquinic acids (DCQAs) and the dicaffeoyltartaric acids (DCTAs) have shown to exhibit anti-viral, in particular anti-HIV activity.

A nucleic acid molecule is "operably linked" when it is placed into a functional relationship with another nucleic acid sequence. For example, a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are covalently linked contiguous. However, enhancers do not have to be contiguous. Linking is accomplished by ligation at convenient restriction sites. If such sites do not exist, synthetic oligonucleotide adapters or linkers are used in accordance with conventional practice.

### Brief description of the drawings

- **Fig. 1:**: Sequential steps of DCQA induction in *Helianthus maximiliani* plant tissue culture by *Sclerotinia sclerotiorum.*
- **Fig. 2:**: Sequential Solid Phase Extraction (SPE) and differential induction of the production of dicaffeoyl derivatives in perennial *Helianthus species (H. maximiliani* Accessions 7 and ACM) inoculation with the Ascomycete *Sclerotinia sclerotiorum* (Lib. De Bary).
- **Fig. 3:**: Flowchart illustrating the method and embodiments of the present invention.

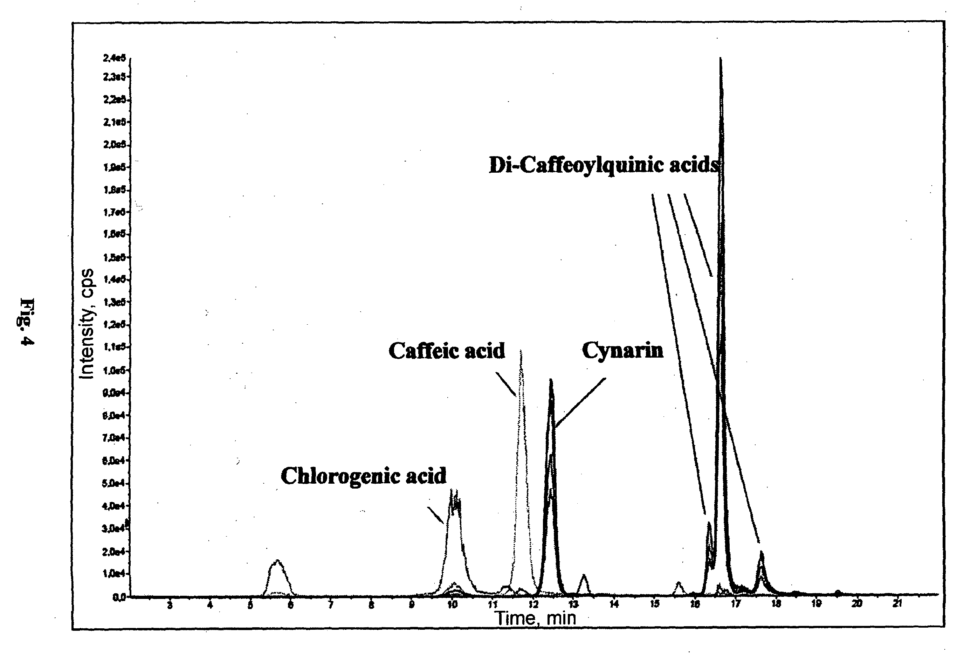

- **Fig. 4:**: LC-MS Chromatogram of DCQAs (Fortification of Cynarin), chlorogenic acid and caffeic acid isolated from stem callus cultures of *Helianthus maximiliani* AC 7 induced by the ascomycete S. *sclerotiorum.*

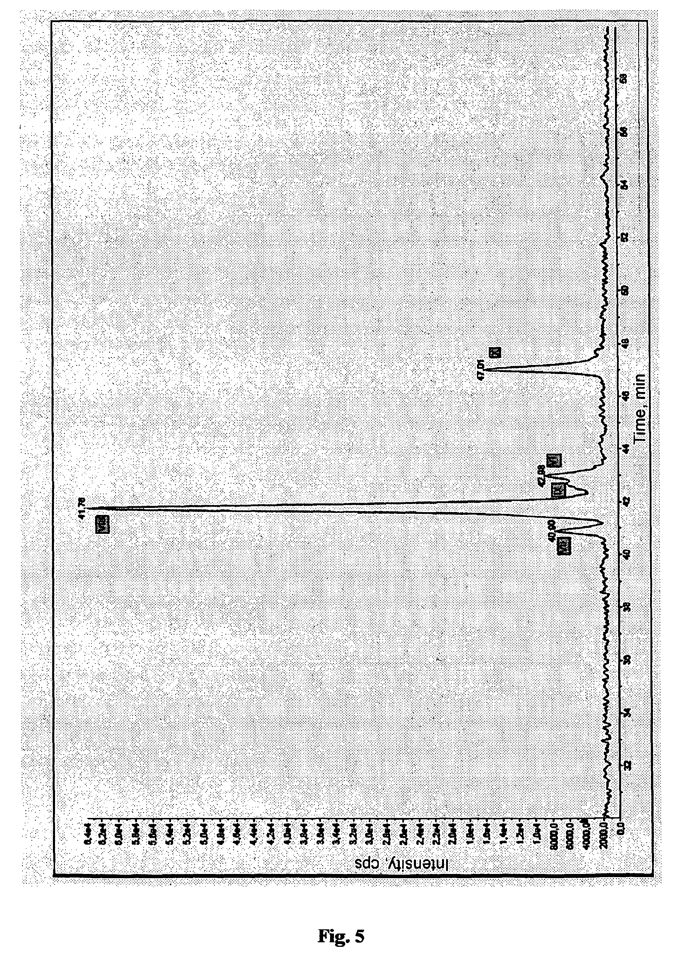

- **Fig. 5:**: LC-MS Chromatogram of DCQA's isolated from stem callus cultures of *H*. *maximiliani* AC 7 induced by the fungus S. *sclerotiorum.* VI 1,4-di-O-Caffeoylquinic acid, VII 1,5-di-O-Caffeoylquinic acid, VIII 3,4-di-O-Caffeoylquinic acid, IX 3,5-di-O-Caffeoylquinic acid, X 4,5-di-O-Caffeoylquinic acid (according to Clifford et al., J. Agric. Food Chem. 53(2005), 3821-3832).

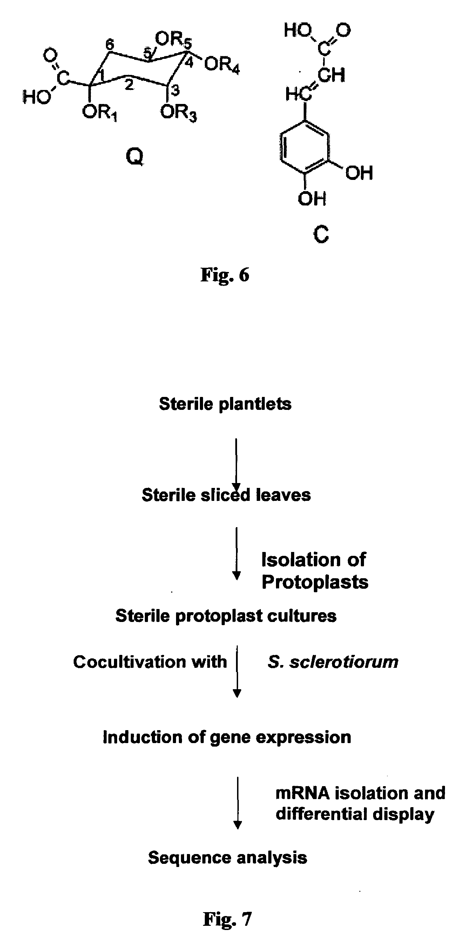

- **Fig. 6:**: Chemical structure of Q: quinic acid and C: caffeic acid R₁₋₅: esterification of caffeoyl-residues
- **Fig. 7:**: Steps for DDRT-PCR analysis of S. sclerotiorum induced mRNA populations of H. maximiliani.
- **Fig. 8:**: Results of DDRT-PCR of treatments showing three examples of differentially expressed fragments of the primers B01 (A) and BO3 (B), respectively. t0: time 0; t10: after 10 min; t30: after 30 min; t60: after 60 min; t24a: after 24 h; t24b: after 24 h, mycelia diluted with mKM-medium (1:100).

### Detailed description of the invention

The present invention relates to methods for increasing the levels of phenolic compounds, in particular depsides in plant cells comprising treating the plant cells with abiotic or biotic stress, most preferably with ascomycetes or elicitors derived thereof to which a given plant or plant cell is susceptible to.

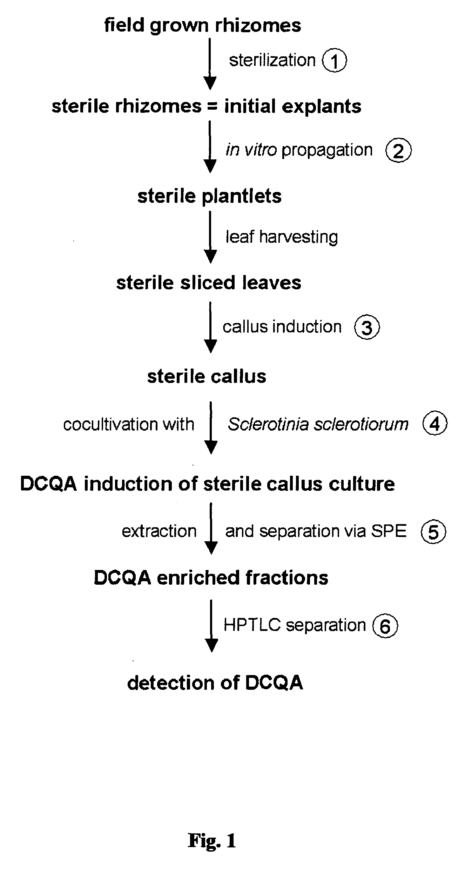

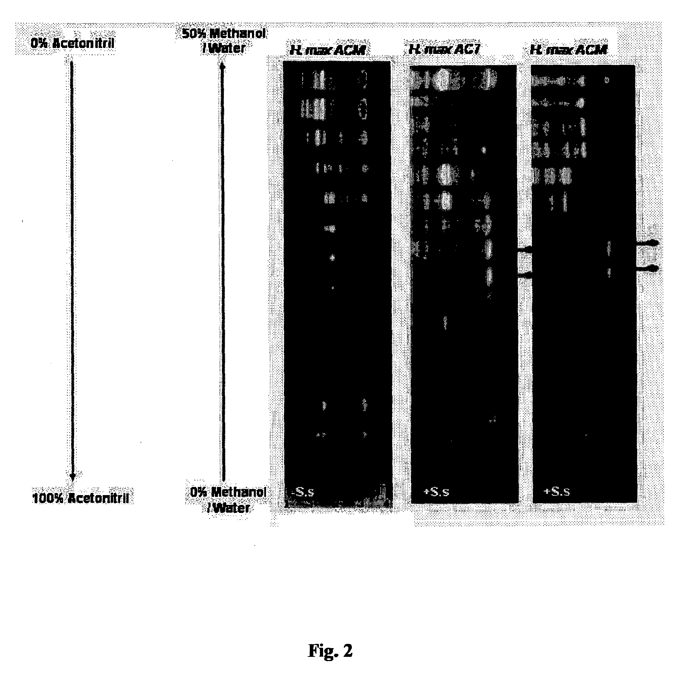

The present invention is based on the observation that differential induction of the production of depsides, in particular of dicaffeoyl derivatives in perennial *Helianthus species,* plants and callus culture can be achieved by subjecting the plant and callus culture, respectively, to biotic stress induced by the phytopathogenic ascomycete *Sclerotinia sclerotiorum* also known as white mold; see the appended Examples and Figures 1 and 2. Thus, experiments performed in accordance with the present invention surprisingly demonstrate that fungi and elicitor derived thereof can be used in order to increase the level of a particular class of phenolic compounds, i.e. depsides which have been shown to be of medical value, especially in the treatment of HIV.

As used herein, the term "depside" refers to a class of compounds which are products of the condensation of two or more molecules of phenolic carboxylic acids, e.g., caffeoyl acid. For example, depsides are esters of polyketide aromatic acids with polyketide phenols such as lecanoric acid. Depsides often carry one or more chlorine substituents; halogenated depsides are indexed separately. For review, see for example Sargent, Prog. Chem. Org. Nat. Comp. 45 (1984), 103 and "Medicinal Natural Products" (Second Edition) published Online 2001, Author: Dewick, Paul M. Online ISBN: 0470846275; ISBN: 0471496405 (2002) John Wiley & Sons, Ltd. Most preferably, the term "depside" as used herein refers to or includes dicaffeoyl derivatives such as preferably dicaffeoylquinic and/or dicaffeoyltartaric acids.

The dicaffeoylquinic acids (DCQAs) and the dicaffeoyltartaric acids (DCTAs) have potent activity against HIV integrase, an essential enzyme that mediates integration of the HIV genome into the host chromosome in vitro, and prevent HIV replication in tissue culture and represent a potentially important class of HIV inhibitors that act at a site distinct from that of current HIV therapeutic agents; see, e.g., McDougall et al., Antimicrob. Agents Chemother. 42 (1998), 140-146.

Identification and characterization of depsides can be performed by methods well known in the art. For example, phenolic acids can be characterized by high-performance liquid chromatography/electrospray ionization mass spectrometry such as exemplified for p-coumaric, caffeic and ferulic acids in black carrots and described in Kammerer et al., Rapid Commun. Mass Spectrom. 18 (2004), 1331-1340.

The step of applying biotic or abiotic stress to a plant cell culture is illustrated in the appended Examples and Figures. Furthermore, methods for how to stress a plant, or corresponding tissue or cell thereof in order to induce the expression of key enzymes, which in turn leads to formation and accumulation of a desired compound or class of compounds is known to the person skilled in the art; see, for example, US patent application No. 2004/0175439 and the references cited below in context with the isolation and characterization of key enzymes and their respective cDNAs. The application of abiotic stress such as drought, salinity, and abscisic acid to sunflower is described in, e.g., Liu and Baird, Am. J. Bot. 91 (2004), 184-191. Furthermore, methods of increasing the levels of flavonoids and phenolic compounds in plants comprising treating the plants with growth-regulating compounds are described in international application WO00/78143. Those methods are also encompassed in the scope of the present invention as far as they are used in accordance with the methods of the present invention for increasing the levels of depsides, in particular dicaffeoylquinic acids (DCQAs) and/or dicaffeoyltartaric acids (DCTAs) as well as dicaffeoyl derivatives in general. The same applies to the teaching of international application WO02/00901 which describes a method for producing fine chemicals, especially vitamin E, vitamin K and/or ubiquinone, by cultivating plants, exhibiting a genetically modified shikimate pathway in relation to the wild type.

In a preferred embodiment of the present invention a biotic stimulus is used, preferably a plant pathogen, for example bacteria or fungi. As demonstrated in the appended examples, said biotic stimulus is most preferably provided by inoculation of the plant and plant cell (callus) culture, respectively, with *Sclerotinia ssp.* such as *Sclerotinia sclerotiorum* or with an elicitor derived thereof.

*Sclerotinia sclerotiorum* is a plant phytopathogenic ascomycete that causes important diseases known as white mold, Sclerotinia wilt or stalk rot, or Sclerotinia head rot on a wide variety of broadleaf crops grown in the North Dakota - Minnesota region. The pathogen is wide spread in the eastern half of North Dakota, especially in the Red River Valley. It is commonly found damaging dry beans, sunflowers, soybeans and canola. There are many other crops in this region that are susceptible such as field pea, potato, mustard, safflower, lentils, flax, borage, crambe, buckwheat, chickpea, lupine, faba bean and numerous vegetables.
There are two other species of Sclerotinia, S. *trifoliorum* and *S. minor. S. trifoliorum* is known on alfalfa and forage legumes, while S. *minor* is primarily a lettuce and peanut pathogen. For review, see for example Index of plant hosts of *Sclerotinia sclerotiorum* by Boland et al., Can. J. Plant Pathol. 16 (1994), 93-100.

Cultures of *Sclerotinia sclerotiorum* can be obtained, maintained and propagated as described in the Example.

Bertinetti and Ugalde describe a method for increasing the activity of phenylalanine-ammonia lyase, PAL in a carrot cell culture by stimulation with a heat-released elicitor (HRE) obtained from *Sclerotinia sclerotiorum* (Bertinetti and Uglade, Mol. Plant Microbe Interact. 9 (1996), 658-663). Thus, Bertinetti and Ugalde do not teach the method of the present invention, i.e. a method for increasing the content of depsides in a plant. Accordingly, the present invention does not relate to the method of inducing PAL in carrot cells as disclosed in the Bertinetti and Ugalde publication. Nevertheless, this document also describes differential display reactions carried out on total RNA extracted from cells exposed for 7 h to the HRE and detection of upregulated genes. Those methods may be also applied in accordance with the methods of the present invention; see also infra.

Mondolot-Cosson and Andary (Acta Horticulturae 381 (1994), 642-645) describe several changes of a *Helianthus* species, *Helianthus resinosus,* as resistance mechanisms after exposure to *Sclerotinia sclerotiorum,* including besides transformation of a bract compound, oxydization of epidermic flavonoids, modification of glandula hair content also an accumulation of caffeic derivatives in stomates, said accumulation of caffeic derivatives being assumed as involved in resistance of the plant to white rot, caused by *Sclerotinia sclerotiorum,* as well as a prevalence of caffeoylaminic compounds, being assumed to be involved in the multiple resistance mechanisms. Accordingly, Mondolot-Cosson and Andary do not describe the increase of the depside concentration in the plants, but rather a reallocation of caffeic compounds. Furthermore, the described effect of accumulation of depsides is explained by the presence of pre-existent defense factors and anatomical and chemical elements, respectively. To the contrary, the teaching of the present invention teaches expressly those compounds not to be pre-formed but rather being inducible and that said inducibility of the responsible genes and enzymes, respectively, as well as of the developed depsides is one of the major criteria.

While *Sclerotinia* or an elicitor derived thereof represent the particularly preferred biotic stimuli to be used in the method of the present invention, other phytopathogenic microorganisms may be used such as bacteria or other fungi, preferably ascomycetes or elicitors derived thereof. Naturally, it is preferred in the method of the present invention to apply the biotic stimuli together with a plant species which is susceptible to the selected stimuli similar as sunflower is susceptible to *Sclerotinia sclerotiorum.*

For review of fungal allergy and pathogenicity see, for example, "Phylogeny and Systematics of the Fungi with Special Reference to the Ascomycota and Basidiomycota" by Prillinger et al. in Breitenbach, Crameri and Lehrer (eds): Fungal Allergy and Pathogenicity. Chem. Immunol. Basel, Karger, 81 (2002), 207-295.

While in principle any plant may be used in accordance with the present invention, which employs the phenylpropanoid pathway, members of the family *Asteraceae, Boraginaceae, Solanaceae, Lamiaceae,* or *Apiaceae* are preferred. Thus, the biotic or abiotic stress for inducing depside production and/or accumulation can be applied to the following plants, but it is also possible to treat plants which are not mentioned: artichoke *(Cynara),* chrysanthemum *(Chrysanthemum),* cornflower *(Centaurea),* coneflower *(Echinacea ssp.),* lettuce *(Lactuca ssp.),* sagebrush, motherwort, mugwort, wormwood, jerusalem artichoke (Artemisia ssp.), and annual and perennial sunflower *(Helianthus ssp.).* Most preferably, the plant is sunflower.

Plants, tissue and cells which have been treated in accordance with the present invention, specifically with *Sclerotinia* or an elicitor derived thereof, in order to increase the content of phenolic compounds, especially depsides, or parts of these plants or products prepared from them (juices, infusions, extracts, fermentation products and fermentation residues) can be used for preparation of curative compositions, health-promoting compositions or tonics for humans and animals, and of cosmetics.

For example, regarding dicaffeoyltartaric acids one of the preferred class of depsides to be increased in plants, tissue and cells in accordance with the methods of the present invention 2,3-O-dicaffeoyltartaric acid (chicoric acid) has been identified to be one of the major constituents in Echinacea, which is responsible to create activity in the immune system by stimulating T-cell production, phagocytosis, lymphocytic activity, cellular respiration, activity against tumor cells and inhibiting hyalurinadase enzyme secretion. Anti-hyaluronidase action is involved in the regeneration of connective tissue destroyed through infection and responsible for the elimination of the pathogenic organism. Macrophages are said to initiate the destruction of pathogens and cancer cells.

Accordingly, the extracts obtained from the mentioned treated plants, tissue and cells may be employed as curative compositions for the adjuvant therapy of chronic inflammatory diseases as well as infectious diseases and cancer.

Furthermore, as mentioned in the background section dicaffeoylquinic acids (DCQAs) and/or dicaffeoyltartaric acids (DCTAs) have been demonstrated to be selective inhibitors of Human Immunodeficiency Virus Type 1 (HIV-1) integrase. Since it could be demonstrated in the appended Examples of the present invention that in *Helianthus* species especially the content of dicaffeoyl derivatives could be increased, said curative compositions are preferably used as pharmaceutical compositions for the treatment of a viral disease, particularly for the treatment of AIDS/HIV.

Hence, the present invention also relates to extracts, juice, and press cakes with an increased content of depsides, obtainable from a plant, tissue or cell as defined and treated in accordance with the present invention as described above. Naturally, the present invention also extends to methods of producing and obtaining depsides comprising isolating said aforedescribed depsides from said extracts, juice, or press cakes. Means and methods for the extraction of phenolic compounds are well known to the person skilled in the art; see also supra. For example, European patent application EP-A-0 299 107 describes processes for separating and obtaining dicaffeoylquinic acids the free acid or salt of the latter from crude vegetable extracts with the aid of gel permeation chromatography on crosslinked modified polysaccharides, in particular on dextrans.

Hitherto, the biochemical pathway in plants, which leads from probably phenylalanine through several branches to the different depsides, in particular to dicaffeoyl derivatives, is not fully understood and remains to be unraveled. However, until to date the elucidation of this particular pathway is hampered by the fact that the phenolic profile of plants is complex and the key enzymatic activities for the synthesis of, for example dicaffeoyl type depsides, have not yet been identified. Furthermore, because of the lack of specific inducers differential screening strategies as employed for the cloning of for example caffeoyl-CoA:3-0-methyltransferase and hydroxycinnamoyl-CoA:tyramine N-(hydroxycinnamoyl)transferase could not be envisaged.

The present invention provides for the first time means and methods for specifically increasing the content of depside compounds in plant cells. In this respect, it is believed that the observed differential induction and increased level of depsides in the plant cells is accompanied with a corresponding differential expression of genes which are involved in the biosynthesis and/or accumulation of these compounds. Accordingly, the present invention also provides for the first time means and methods for identifying and isolating the key enzymes and their encoding nucleic acid molecules, which are involved in the biosynthesis of particular depside compounds in vivo.

Hence, in one embodiment the present invention relates to a method of identifying and obtaining an enzyme correlating with or involved in the biosynthesis or accumulation of depsides in a plant, said method comprising
(a) providing an extract of plant, tissue or cell as defined above, said plant, tissue or cell having been treated with a plant pathogen such as *Sclerotinia* or an elicitor derived thereof; and
(b) isolating a protein which is more abundant in said extract compared to an extract of a non-treated control plant, plant tissue or cell, or identifying or isolating a peptide fragment corresponding to said protein.

A corresponding approach for the purification of a protein hydrolyzing hydroxycinnamoyl-CoA esters from tobacco stem extracts by a series of high pressure liquid chromatography steps is described in Hoffmann et al., J. Biol. Chem. 278 (2003), 95-103. Subsequent determination of its N-terminal amino acid sequence allowed design of primers permitting the corresponding cDNA to be cloned by PCR. The cDNA was expressed in bacterial cells as a recombinant protein fused to glutathione S-transferase. The fusion protein was affinity-purified and cleaved to yield the recombinant enzyme for use in the study of catalytic properties. The enzyme catalyzed the synthesis of shikimate and quinate esters shown recently to be substrates of the cytochrome P450 3-hydroxylase involved in phenylpropanoid biosynthesis. It was shown that p-coumaroyl-CoA and caffeoyl-CoA are the best acyl group donors and that the acyl group is transferred more efficiently to shikimate than to quinate. The enzyme also catalyzed the reverse reaction, i.e. the formation of caffeoyl-CoA from chlorogenate (5-O-caffeoyl quinate ester).
Corresponding experiments for the molecular cloning and induction of caffeoyl-CoA 3-O-methyltransferase involving treatment of cultured parsley cells with a crude elicitor have been described by Schmitt et al., J. Biol. Chem. 266 (1991), 17416-17423.

Of course, the mentioned enzymes and methods may also be used in any one of the processes described below for the production of depsides with recombinant enzymes and/or genetically engineered cells which express said enzyme(s).

In a further embodiment, the present invention relates to a method of identifying and obtaining a nucleic acid molecule correlating with or involved in the biosynthesis or accumulation of depsides in a plant, said method comprising
(a) providing a nucleic acid sample of plant, tissue or cell as defined above, said plant, tissue or cell having been treated with a plant pathogen such as *Sclerotinia* or an elicitor derived thereof;
(b) identifying, and optionally isolating a nucleic acid molecule which is differentially expressed in said nucleic acid sample compared to a nucleic acid sample of a non-treated control plant, plant tissue or cell.

Usually, step (b) of the method of the invention comprises subjecting at least one of said RNA pools to cDNA synthesis. Methods of cDNA synthesis are well-known to the person skilled in the art; see Berger, Sambrook and Ausubel (all supra) but also the literature cited before the experimental and the examples themselves.

In one preferred embodiment of the method of the present invention after step (b) said nucleic acid, e.g. RNA or resulted cDNA is contacted with nucleic acid primers and an effective amount of reagents necessary for performing an amplification reaction. In vitro amplification techniques are well known in the art. Examples of techniques sufficient to direct persons of skill through such in vitro methods, including the preferred polymerase chain reaction (PCR), but also the ligase chain reaction (LCR), Q-replicase amplification and other RNA polymerase mediated techniques (e.g., NASBA), are found in Berger, Sambrook and Ausubel (all supra) as well as in US patent No. 4,683,202; PCR Protocols, A Guide to Methods and Applications (Innis et al., eds.) Academic Press Inc., San Diego Academic Press, Inc. San Diego, CA (1990) (Innis); Arnheim & Levinson (October 1, 1990) C & EN 36-47; The Journal OfNIH Research (1991) 3, 81-94; Kwoh et al., Proc. Natl. Acad. Sci. USA 86 (1989), 1173; Guatelli et al., Proc. Natl. Acad. Sci. USA 87 (1990), 1874; Lomell et al., J. Clin. Chem. 35 (1989), 1826; Landegren et al., Science 241 (1988), 1077-1080; Van Brunt, Biotechnology 8 (1990), 291-294; Wu and Wallace, Gene 4 (1989), 560; Barringer et al., Gene 89 (1990), 117; and Sooknanan and Malek, Biotechnology 13 (1995), 563-564.

Improved methods of cloning of in vitro amplified nucleic acids are described in US patent No. 5,426,039. Improved methods of amplifying large nucleic acids by PCR are summarized in Cheng et al., Nature 369 (1994), 684, and the references therein, in which PCR amplicons of up to 40kb are generated. A variety of DNA polymerases can be used during PCR with the subject invention. Preferably, the polymerase is a thermostable DNA polymerase such as may be obtained from a variety of bacterial species, including Thermus aquaticus (Taq), Thermus thermophilus (Tth), Thermus filiformis, Thermus flavus, Thermococcus literalis, and Pyrococcus furiosus (Pfu). Many of these polymerases may be isolated from the bacterium itself or obtained commercially. Polymerases to be used with the subject invention can also be obtained from cells which express high levels of the cloned genes encoding the polymerase.

The subject invention can also be used with long distance (LD) PCR technology (Barnes, Proc. Natl. Acad. Sci. USA 91 (1994), 2216-2220; Cheng et al., Proc. Natl. Acad. Sci. USA 91 (1994), 5695-5699). LD PCR, which uses a combination of thermostable DNA polymerases, produces much longer PCR products with increased fidelity to the original template as compared to conventional PCR performed using Taq DNA polymerase alone.

One of skill will appreciate that essentially any RNA can be converted into a single- or double stranded DNA suitable for restriction digestion, PCR expansion and/or sequencing using reverse transcriptase and a polymerase; see Ausubel, Sambrook and Berger, all supra. Oligonucleotides for use as primers, e.g., in sequencing, amplification reactions and for use as nucleic acid sequence probes are typically synthesized chemically according to the solid phase phosphoramidite triester method described by Beaucage and Caruthers, Tetrahedron Lett. 22 (1981), 1859, or can simply be ordered commercially.

The method of the present invention may be performed in accordance with the experiments described in Back et al., Plant Cell Physiol. 42 (2001), 475-481, for the cloning and characterization of a hydroxycinnamoyl-CoA:tyramine N-(hydroxycinnamoyl)transferase (THT), a pivotal enzyme in the synthesis of N-(hydroxycinnamoyl)-amines. In those experiments, induction of expression has been achieved by applying UV-C to and wounding of *Capsicum annuum* (hot pepper) and for the isolation of the target cDNA a differential screening strategy was used. The recombinant pepper THT enzyme was purified using a bacterial overexpression system. The purified enzyme has a broad substrate specificity including acyl donors such as cinnamoyl-, sinapoyl-, feruloyl-, caffeoyl-, and 4-coumaroyl-CoA and acceptors such as tyramine and octopamine. Hence, similar set ups can also be used for the recombinant expression and characterization of enzymes identified in accordance with the present invention.

Likewise, a cDNA clone encoding a chalcone synthase (designated HvCHS2) with an unusual substrate preference was isolated by differential hybridization from a library prepared from barley leaves inoculated with the fungus *Blumeria graminis f.sp. hordei* (Bgh); see Christensen et al., Plant Mol. Biol. 37 (1998), 849-857. To compare the activity of HvCHS2 with the activity of barley naringenin-CHS (CHS1), the two enzymes were expressed in *Escherichia coli.* Both HvCHS2 and CHS1 catalyze the formation of chalcones. However, HvCHS2 and CHS1 differ in their substrate requirements. CHS1 uses cinnamoyl-CoA and 4-coumaroyl-CoA at comparable rates whereas feruloyl-CoA is a poor substrate for this enzyme. In contrast, HvCHS2 converts feruloyl-CoA and caffeoyl-CoA at the highest rate whereas cinnamoyl-CoA is a poor substrate.

In a preferred embodiment, the method of the present invention is performed with a plant species as described above, preferably said plant is a member of the family *Asteraceae.* Most preferably, said plant is sunflower. In this context, it is referred to a similar approach for the identification of a novel gene, HAABRC5, from *Helianthus annuus (Asteraceae)* using differential display of mRNA transcripts isolated from cells upon exposure to high salinity as has been described in Liu and Baird, Am. J. Bot. 91 (2004), 184-191.

In the present case, however, it is intended to identify and isolate a gene or cDNA involved in the biosynthesis of phenolic compounds, i.e. depsides and preferably dicaffeoyl derivatives as mentioned above. Without intending to be bound by theory it is believed that one major class of genes involved in the biosyntheses or accumulation of depsides encode enzymes, particularly enzymes which exhibit transferase activity.

In one embodiment, the method of the invention includes suppression subtractive hybridization (SSH). This most recently developed method usually deploys several hybridization steps to compare two nucleic acid populations of for example differentially induced cells (driver and tester) after they were enriched by PCR.
The principle behind this technique is a two-step hybridization with an excess of nucleic acid from a "driver" sample (i.e. sample of untreated plants, plant tissue or cells) compared with that from a "tester" sample (i.e. sample of plants, plant tissue or cells which have been treated to increase the content of depsides). After re-annealing tester and driver nucleic acid, only specific nucleic acid fragments (tester nucleic acid) with an appropriate pair of adaptors can participate in an exponential PCR amplification when a defined oligonucleotide is used as primer. Next, a secondary PCR amplification is performed using nested primers to further reduce background PCR products and enrich for tester-specific sequences.

Methods of suppression subtractive hybridization (SSH) are known to the person skilled in the art and are described, for example, in Diatchenko et al., Meth. Enzym. 303 (1999), 349-380 and international applications WO0̅3/093501, WO98/49345 and WO96/23079, the disclosure contents of which are incorporated herein by reference.

Design of adapters, for use in SSH technique, and primers as well as the choice of appropriate hybridization conditions can be performed according to known methods, see, e.g., Nucleic Acid Hybridization (1985) Ed. James, B. D. & Higins, S. J. (IRL Press Ltd., Oxford); Lukyanov et al., Bioorganic Chem. (Russian) 20 (1994), 701-704; Siebert et al., Nucleic Acids Res. 23 (1995), 1087-1088; Clontech PCR-Select cDNA Subtraction Kit 7 rxns K1804-1; PCR-Select Differential Screening Kit each K1808-1; Custom Clontech PCR-Select Subtraction: Level I each CS1103; Custom Clontech PCR-Select Subtraction Differential Screening: Level II each CS1104; Custom Clontech PCR-Select SMART Amplification CS 1105 (Clontech 1020 East Meadow Circle Palo Alto, CA 94303-4230 USA).

The adapters and primers used in the subject invention can be readily prepared by the person skilled in the art using a variety of techniques and procedures. For example, adapters and primers can be synthesized using a DNA or RNA synthesizer. In addition, adapters and primers may be obtained from a biological source, such as through a restriction enzyme digestion of isolated DNA. The primers can be either single- or double-stranded. Preferably, the primers are single stranded. In a particular preferred embodiment of the methods of the present invention, said adapters or nucleic acid primers comprise a nucleotide sequence comprising a restriction endonuclease recognition site.

In a further embodiment, the method of the present invention comprises the step of cloning and/or sequencing the identified differentially expressed nucleic acid molecules. Detailed descriptions of conventional methods, such as those employed in sequencing, the construction of vectors and plasmids, the insertion of genes encoding polypeptides or the corresponding antisense construct into such vectors and plasmids, the introduction of plasmids into host cells, and the expression and determination of genes and gene products can be obtained from numerous publications, including Sambrook et al., (1989) Molecular Cloning: A Laboratory Manual, 2nd ed., Cold Spring Harbor Laboratory Press. In particular, differential transcripts can be isolated as the corresponding bands in a PAGE according to standard protocols.

Candidate nucleic acids or encoded polypeptides identified in such a manner can be validated by expressing them and observing the phenotype or using them as marker. A further embodiment of the method therefore comprises the over-expression or inhibition of expression of the identified candidate nucleic acid or encoded polypeptide in said cell, tissue or organism, preferably a plant, for their capability of inducing a responsive change in the phenotype of said cell, tissue or organism, wherein said phenotype is preferably related to the level of depsides.

In a still further embodiment the method of the present invention further comprises using the identified, sequenced and/or cloned nucleic acid molecule, i.e. cDNA or fragment thereof or a primer derived from the amino acid sequence of the identified protein or peptide as a probe or source of primers for cloning the corresponding gene or full length cDNA. Methods which are well known to those skilled in the art can be used to obtain and probe genomic or cDNA libraries; see, for example, the techniques described in Sambrook, Molecular Cloning: A Laboratory Manual, Cold Spring Harbor Laboratory (1989) N.Y. and Ausubel, Current Protocols in Molecular Biology, Green Publishing Associates and Wiley Interscience, N.Y. (1994). Furthermore, various DNA libraries are commercially available; see, e.g., Clontech.
A corresponding experimental approach for the molecular cloning of caffeoyl-CoA 3-O-methyltransferase has been described by Schmitt et al., J. Biol. Chem. 266 (1991), 17416-17423; see also supra.

The successful cloning of a nucleic acid molecule involved in the biosynthesis of depsides can be determined by methods well known in the art. For example, the cloned nucleic acid molecule may be preliminarily evaluated by overexpression or suppression of the target gene in plant cells via, e.g., the transient expression as sense or antisense, optionally together with a "reporter" gene; see also infra. A reporter gene is a component on the expression vector introduced into the cell, or a component of a separate DNA construct which is co-introduced into the cell along with the DNA construct comprising the transgene. The property conferred on the transformed cell or tissue by the introduction of the reporter gene is usually easily detectable (e.g., expression of an easily assayable enzyme). "Transient expression" denotes the expression of a gene before the gene has been stably integrated into the genome of the treated cells or tissue. For example, commonly used reporter genes are the genes coding for the production of chloramphenicol acetyltransferase, which confers resistance to the antibiotic chloramphenicol, or the *E.coli* [beta]-glucuronidase gene (gusA), the products of which can be detected by a histochemical assay.

Optionally, the transformed plant host cells are used to regenerate transgenic plants. In plants, every cell is capable of regenerating into a mature plant and, in addition, contributing to the germ line such that subsequent generations of the plant will contain the transgene. Growth of transformed plant cells and regeneration of such cells into mature plants is routine among those skilled in the art; see also infra.

If the cloned nucleic acid molecule is effectively involved in the biosynthesis of depsides, the level of these compounds in the mentioned transiently or stably genetically modified plant cells or plants will be different compared to corresponding wild type plant cells and plants, respectively, in particular when treated in accordance with the method of the present invention with abiotic or biotic stress; see supra.

Depending on the observed phenotype it will be immediately evident whether the cloned nucleic acid molecule is positively, negatively or involved at all in the biosynthesis and/or accumulation of depsides in plant cells.

The promoters and other regulatory sequences of the identified cDNAs may be obtained from genomic DNA by using polymerase chain reaction (PCR), and then cloned into the construct. Standard recombinant DNA and molecular cloning techniques used here are well known in the art and are described by Sambrook et al., (1989) and by Silhavy et al, Experiments with Gene Fusions, Cold Spring Harbor Laboratory, Cold Spring Harbor, N.Y. (1984); see also supra and infra.

Accordingly, the present invention also relates to the nucleic acid molecule obtainable by a method of the invention as described herein. Thus, the present invention relates to nucleic acid molecules encoding an enzyme involved in the biosynthesis or accumulation of depsides in a plant comprising a nucleotide sequence selected from the group consisting of:
(a) a nucleotide sequence encoding an amino acid sequence which is at least 70% identical to an amino acid sequence encoded by the initially identified nucleic acid molecule of the present invention;
(b) a nucleotide sequence of at least 15 nucleotides in length which hybridizes under stringent conditions to such a nucleic acid molecule;
(c) a nucleotide sequence which represents a fragment, derivative or allelic variation of a nucleotide sequence specified (a) or (b); and
(d) a nucleotide sequence obtainable by probing a cDNA or genomic library of a plant species of the family *Asteraceae,* preferably sunflower, with a nucleic acid probe or primers comprising a nucleotide sequence of any one of (a) to (c); or
a nucleic acid molecule comprising a nucleotide sequence complementary to a nucleotide sequence of any one of (a) to (d).

In one preferred embodiment the present invention relates to a nucleic acid molecule comprising the nucleotide sequence depicted in SEQ ID NO: 1 or a selectively hybridizing sequence; see supra.

Nucleic acid molecules according to the present invention may be provided isolated and/or purified from their natural environment, in substantially pure or homogeneous form, or free or substantially free of other nucleic acids of the species of origin. Where used herein, the term isolated encompasses all of these possibilities. The nucleic acid molecules may be wholly or partially synthetic. In particular they may be recombinant in that nucleic acid sequences which are not found together in nature (do not run contiguously) have been ligated or otherwise combined artificially. Alternatively, they may have been synthesized directly e.g. using an automated synthesizer. Most preferably the nucleic acid is derived from sunflower.

In addition to the hybridization methods described above, homologs of the initially isolated nucleic acid molecules and genes isolated in accordance with the present invention can be identified in silico using any of a variety of sequence alignment and comparison protocols. For the purposes of the ensuing discussion, the following terms are used to describe the sequence relationships between a marker nucleotide sequence and a reference polynucleotide sequence: A "reference sequence" is a defined sequence used as a basis for sequence comparison with a test sequence, e.g., a candidate marker homolog, of the present invention. A reference sequence may be a subsequence or the entirety of a specified sequence, for example, a segment of a full-length cDNA or gene sequence, or the complete cDNA or gene sequence; see also the Examples. The same rationale applies to reference amino acid sequences for sequence comparison of amino acid sequences.

Methods of alignment of sequences for comparison are well-known in the art. Optimal alignment of sequences for comparison may be conducted by the local homology algorithm of Smith and Waterman, Adv. Appl. Math. 2 (1981), 482; by the homology alignment algorithm of Needleman and Wunsch, J. Mol. Biol. 48 (1970), 443; by the search for similarity method of Pearson and Lipman, Proc. Natl. Acad. Sci. USA 85 (1988), 2444; by computerized implementations of these algorithms, including, but not limited to: CLUSTAL in the PC/Gene program by Intelligenetics, Mountain View, California; GAP, BESTFIT, BLAST, FASTA, and TFASTA in the Wisconsin Genetics Software Package, Genetics Computer Group (GCG), 575 Science Dr., Madison, Wisconsin, USA; the CLUSTAL program is well described by Higgins and Sharp, Gene 73 (1988), 237-244; Higgins and Sharp, CABIOS 5 (1989), 151-153; Corpet et al., Nucleic Acids Res. 16 (1988), 10881-90; Huang et .al., Computer Applications in the Biosciences 8 (1992), 155-65, and Pearson et al., Methods in Molecular Biology 24 (1994), 307331.

The BLAST family of programs which can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences; see, e.g., Current Protocols in Molecular Biology, Chapter 19, Ausubel et al., Eds., (1995) Greene Publishing and Wiley-Interscience, New York; Altschul et al., J. Mol. Biol. 215 (1990), 403-410; and, Altschul et al., Nucleic Acids Res. 25 (1997), 3389-3402.

Software for performing BLAST analyses is publicly available, e.g., through the National Center for Biotechnology Information (NCBI). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are then extended in both directions along each sequence for as far as the cumulative alignment score can be increased.

GAP (Global Alignment Program) can also be used to compare a polynucleotide or polypeptide of the present invention with a reference sequence. GAP uses the algorithm of Needleman and Wunsch, J. Mol. Biol. 48 (1970), 443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 100. Thus, for example, the gap creation and gap extension penalties can each independently be: 0, 1, 2, 3, 4, 5, 6, 7, 8, 9, 10 15, 20, 30, 40, 50, 60 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62; see, e.g., Henikoff & Henikoff, Proc. Natl. Acad. Sci. USA 89 (1989), 10915.

The percentage sequence identity of a homologous gene or protein to its reference sequence (e.g., any nucleic acid molecule identified according to the method of the present invention or its encoded amino acid sequence) is typically at least 70% and, rounded upwards to the nearest integer, can be expressed as an integer selected from the group of integers between 70 % and 99 %. Thus, for example, the percentage sequence identity to a reference sequence is preferably 70 %, 80%, 85%, 90%, 95%, 97%, or 99%. As mentioned, sequence identity can be calculated using, for example, the BLAST, CLUSTALW, or GAP algorithms under default conditions.

Again, and without intended to be bound by theory it is expected that the nucleic acid molecules identified in accordance with the present invention encode enzymes, wherein said enzymes are involved in primary phenolic metabolism and shikimi acid metabolism in plants, and/or have high substrate affinity for polycarbonic, polyhydroxy acids and/or phenolic acids. The cloning of a gene encoding an enzyme, i.e. hydroxycinnamoyl-CoA quinate hydroxycinnamoyl transferase (HQT) which is said to be involved in chlorogenic acid synthesis has been described in international application WO2004/001028, the disclosure content of which is incorporated herein by reference. Hence, most of the embodiments described therein in relation to HQT, in particular with respect to expression vectors, transformation and regeneration of transgenic plants may be adapted to the embodiments of the present invention.

As mentioned before, dicaffeoylquinic and dicaffeoyltartaric acids have been shown to be potent inhibitors of HIV-1 integrase and replication. Furthermore, analogues of the dicaffeoylquinic and dicaffeoyltartaric acids have recently been shown to be potent inhibitors of HIV infection as well; see King et al., J. Med. Chem. 42 (1999), 497-509 and for review Gupta and Nagappa, Curr. Med. Chem. 10 (2003), 1779-1794. While it could be shown that these analogues of the DCTAs and the DCQAs can be synthesized in vitro via conventional organic chemistry (see, e.g., international application W099/48371), it nevertheless would be desirable to provide alternative means, in particular for the large scale production of such compounds.

In accordance with the present invention this may be achieved by providing the nucleic acid molecules and their encoded enzymes identified by the methods described herein. However, it may be that the naturally occurring enzymes do not allow the biochemical production of the full spectrum of desired depsides, for example dicaffeoyl derivatives and analogs. If so, the provision of the nucleic acid molecules of the present invention allow the making of variant enzymes, wherein the enzymatic activity and/or substrate or product specificity of the enzyme encoded by the originally identified and cloned nucleic acid molecule has been altered.

Artificial variants (derivatives) may be prepared by those skilled in the art, for instance by site directed or random mutagenesis, or by direct synthesis. Preferably, the variant nucleic acid molecule is generated either directly or indirectly (e.g. via one or more amplification or replication steps) from an original nucleic acid molecule having all or part of the original sequence.

Such functional variants have an altered sequence in which one or more of the amino acids are deleted or substituted, or one or more amino acids are inserted, as compared to the reference amino acid sequence, i.e. the amino acid sequence of the given wild type enzyme. Sequences which are at least 95% identical have no more than 5 alterations, i.e. any combination of deletions, insertions or substitutions, per 100 amino acids of the reference amino acid sequence. Percent identity may be determined by comparing the amino acid sequence of the functional variant with the reference sequence using MEGALIGN project in the DNA STAR program. The variant sequences and reference sequences are aligned for identity calculations using the method of the software basic local alignment search tool in the BLAST network service (the National Center for Biotechnology Information, Bethesda, MD) which employs the method of Altschul et al., Nucleic Acid Res. 25 (1997), 3389-3402. Identities are calculated, for example, by the Align program (DNAstar, Inc.). In all cases, internal gaps and amino acid insertions in the candidate sequence as aligned are not ignored when making the identity calculation. Preferably, the substitutions, deletions, or additions are made at the positions in the catalytic center of the respective enzyme.

While functional variants of the initially cloned wild type enzyme may have non-conservative amino acid substitutions, it is preferred that the functional variant have the conservative amino acid substitutions. In conservative amino acid substitutions, the substituted amino acid has similar structural or chemical properties with the corresponding amino acid in the reference sequence. By way of example, conservative amino acid substitutions involve substitution of one aliphatic or hydrophobic amino acids, e.g. alanine, valine, leucine and isoleucine, with another; substitution of one hydroxyl-containing amino acid, e.g. serine and threonine, with another; substitution of one acidic residue, e.g. glutamic acid or aspartic acid, with another; replacement of one amide-containing residue, e.g. asparagine and glutamine, with another; replacement of one aromatic residue, e.g. phenylalanine and tyrosine, with another; replacement of one basic residue, e.g. lysine, arginine and histidine, with another; and replacement of one small amino acid, e.g., alanine, serine, threonine, methionine, and glycine, with another.

The present invention also relates to the above described nucleic acid molecules which are detectably labeled. Such nucleic acid probes can be cloned and/or synthesized. Detectable labels suitable for use with nucleic acid probes include any composition detectable by spectroscopic, radioisotopic, photochemical, biochemical, immunochemical, electrical, optical or chemical means. Useful labels include biotin for staining with labeled streptavidin conjugate, magnetic beads, fluorescent dyes, radiolabels, enzymes, and colorimetric labels. Other labels include ligands which bind to antibodies labeled with fluorophores, chemiluminescent agents, and enzymes. Labeling markers is readily achieved such as by the use of labeled PCR primers to marker loci. The hybridized probe is then detected most typically by autoradiography or other similar detection technique (e.g., fluorography, liquid scintillation counter, etc.). Examples of specific hybridization protocols are widely available in the art; see, e.g., Berger, Sambrook, Ausubel, all supra.

It will be appreciated that numerous vectors are available in the art for the isolation and replication of the nucleic acids of the invention. For example, plasmids, cosmids and phage vectors are well known in the art, and are sufficient for many applications.

Hence, in a further aspect of the present invention, the nucleic acid molecules are in the form of a recombinant and preferably replicable vector. Vectors are defined to include, inter alia, any plasmid, cosmid, phage or Agrobacterium binary vector in double or single stranded linear or circular form which may or may not be self transmissible or mobilizable, and which can transform a prokaryotic or eukaryotic host either by integration into the cellular genome or exist extrachromosomally (e.g. autonomous replicating plasmid with an origin of replication).

Generally speaking, those skilled in the art are well able to construct vectors and design protocols for recombinant gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: A Laboratory Manual: 2nd edition, Sambrook et al, 1989, Cold Spring Harbor Laboratory Press or Current Protocols in Molecular Biology, Second Edition, Ausubel et al. eds., John Wiley & Sons, 1992; see also supra.

Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells). A vector including a nucleic acid according to the present invention does not need to include a promoter or other regulatory sequence, particularly if the vector is to be used to introduce the nucleic acid into cells for recombination into the genome.

Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

By "promoter" is meant a sequence of nucleotides from which transcription of DNA may be initiated, operably linked downstream (i.e. in the 3'-direction on the sense strand of double-stranded DNA). In a preferred embodiment, the promoter is an inducible promoter. The term "inducible" as applied to a promoter is well understood by those skilled in the art. In essence, expression under the control of an inducible promoter is "switched on" or increased in response to an applied stimulus. The nature of the stimulus varies between promoters. Some inducible promoters cause little or undetectable levels of expression (or no expression) in the expression in the absence of the stimulus. Whatever the level of expression is in the absence of the stimulus, expression from any inducible promoter is increased in the presence of the correct stimulus.

Thus, this aspect of the invention provides a gene construct, preferably a replicable vector, comprising a promoter (optionally inducible) operably linked to a nucleotide sequence provided by the present invention, such promoter comprising regulatory elements permitting expression in prokaryotic or eukaryotic host cells.

Particularly of interest in the present context are nucleic acid constructs which operate as plant vectors. Specific procedures and vectors previously used with wide success upon plants are described by Guerineau and Mullineaux (1993) (Plant transformation and expression vectors. In: Plant Molecular Biology Labfax (Croy RRD ed) Oxford, BIOS Scientific Publishers, pp 121-148). Suitable vectors may include plant viral-derived vectors; see, e.g., European patent application EP-A-0 194 809. Suitable promoters which operate in plants include the Cauliflower Mosaic Virus 35S (CaMV 35S). Other examples are disclosed at pg 120 of Lindsey & Jones (1989) Plant Biotechnology in Agriculture Pub. Press, Milton Keynes, UK. The promoter may be selected to include one or more sequence motifs or elements conferring developmental and/or tissue-specific regulatory control of expression. Inducible plant promoters include the ethanol induced promoter of Caddick et al, Nature Biotechnology 16 (1998), 177-180.

If desired, selectable genetic markers may be included in the construct, such as those that confer selectable phenotypes such as resistance to antibiotics or herbicides (e.g. kanamycin, hygromycin, phosphinotricin, chlorsulfuron, methotrexate, gentamycin, spectinomycin, imidazolinones and glyphosate).

Hence, the present invention also relates to the production of transgenic organisms, which can be bacteria, yeast, fungi, plants, or animals, transduced with the nucleic acid molecules, e.g., cloned cDNAs, genes or vectors of the invention. A thorough discussion of techniques relevant to bacteria, unicellular eukaryotes and cell culture can be found in references enumerated above and are briefly outlined as follows. Several well-known methods of introducing target nucleic acids into bacterial cells are available, any of which can be used in the present invention. These include: fusion of the recipient cells with bacterial protoplasts containing the DNA, electroporation, projectile bombardment, and infection with viral vectors (discussed further, below), etc. Bacterial cells can be used to amplify the number of plasmids containing DNA constructs of this invention. The bacteria are grown to log phase and the plasmids within the bacteria can be isolated by a variety of methods known in the art (see, for instance, Sambrook et al.). In addition, a plethora of kits are commercially available for the purification of plasmids from bacteria. For their proper use, follow the manufacturer's instructions (see, for example, EasyPrepTM, FlexiPrepTM, both from Pharmacia Biotech; StrataCleanTM, from Stratagene, and QIAprepTM from Qiagen). The isolated and purified plasmids are then further manipulated to produce other plasmids, used to transfect, for example, plant cells or incorporated into *Agrobacterium tumefaciens* related vectors to infect plants. Typical vectors contain transcription and translation terminators, transcription and translation initiation sequences, and promoters useful for regulation of the expression of the particular target nucleic acid.

The vectors optionally comprise generic expression cassettes containing at least one independent terminator sequence, sequences permitting replication of the cassette in eukaryotes, or prokaryotes, or both, (e.g., shuttle vectors) and selection markers for both prokaryotic and eukaryotic systems. Vectors are suitable for replication and integration in prokaryotes, eukaryotes, or preferably both; see, e.g., Giliman & Smith, Gene 8 (1979), 81; Roberts et al., Nature 328 (1987), 731; Schneider et al., Protein Expr. Purif. 10 (1995), 6435; Ausubel, Sambrook, Berger (all supra). A catalogue of Bacteria and Bacteriophages useful for cloning is provided, e.g., by the ATCC, e.g., The ATCC Catalogue of Bacteria and Bacteriophage (1992) Gherna et al. (eds) published by the ATCC. Additional basic procedures for sequencing, cloning and other aspects of molecular biology and underlying theoretical considerations are also found in Watson et al. (1992) Recombinant DNA, Second Edition, Scientific American Books, NY.

In a further embodiment the present invention relates to a method of producing an enzyme involved in the biosynthesis or accumulation of depsides comprising
(a) culturing the host cell of the present invention under conditions allowing expression of the nucleic acid molecule; and
(b) isolating the enzyme from the cell or cell culture.

Hence, the present invention also encompasses the expression product of any of the coding (sense) nucleic acid sequences disclosed above, and methods of making the expression product by expression from encoding nucleic acid therefore under suitable conditions, which may be in suitable host cells. Following expression, the product may be isolated from the expression system (e.g. microbial) and may be used as desired. For the purpose of easy production of depsides in vitro the polypeptide is preferably a transferase.

As already explained above, in one embodiment the activity and/or substrate or product specificity of the wild type polypeptide may be altered compared to the wild type enzyme. For doing so, the enzymatic activity may be altered by modification of the underlying coding sequence, see also supra, or by posttranslational modifications, for example determined by the chosen host and/or culture conditions. Furthermore, it is possible to chemically modify the expressed and purified enzyme; see for example modification of L-asparaginase with colominic acid and the new characteristics of the modified enzyme described in Wang et al., Sheng Wu Gong Cheng Xue Bao 16 (2000), 517-520, see also Lee et al., J. Biol. Chem. 276 (2001), 14804-14813.

The purified polypeptides or fragment thereof, produced recombinantly by expression from encoding nucleic acid may be used to raise antibodies employing techniques which are standard in the art; see, e.g., in Harlow and Lane "Antibodies, A Laboratory Manual", CSH Press, Cold Spring Harbor, 1988. Methods of producing antibodies include immunizing a mammal (e.g. mouse, rat, rabbit, horse, goat, sheep or monkey) with the protein or a fragment thereof. Antibodies may be obtained from immunized animals using any of a variety of techniques known in the art, and might be screened, preferably using binding of antibody to antigen of interest. For instance, Western blotting techniques or immunoprecipitation may be used; see, e.g., Armitage et al, Nature 357 (1992), 80-82. Antibodies may be polyclonal or monoclonal. As an alternative or supplement to immunizing a mammal, antibodies with appropriate binding specificity may be obtained from a recombinantly produced library of expressed immunoglobulin variable domains, e.g. using lambda bacteriophage or filamentous bacteriophage which display functional immunoglobulin binding domains on their surfaces; for instance see international application W092/01047.

Specific binding members such as antibodies and polypeptides including antigen binding domains of antibodies that bind and are preferably specific for the polypeptides of the present invention represent further aspects of the present invention, as do their use and methods which employ them.

The invention therefore relates to the use of the above-described nucleic acid molecules, of the above-described vectors, in particular the expression cassettes for generating genetically modified plants or for the transformation of plants, plant cells, plant tissues or plant parts. The preferred purpose of the use is to increase the depside content of the plant or the plant parts. Hence, in a further embodiment the present invention relates to a method of producing a transgenic plant comprising introducing into a cell a nucleic acid molecule or a vector as described above. Preferably, the genetic modification leads to a change in the content of a depside compared to the wild type plant.

By "transgenic plant" it is preferably meant that this plant or tissue or fruits, etc. thereof comprises transgenic plant cells which contain stably integrated into the genome at least one recombinant nucleic acid molecule which is either heterologous with respect to the transgenic plant and normally not present in non-transformed cells or which is homologous with respect to the transgenic plant but located in a different genomic environment in the transgenic cells in comparison to wild-type cells.

The term "heterologous" is used broadly in this aspect to indicate that the gene/sequence of nucleotides in question (e.g. cloned nucleic acid molecule encoding an enzyme involved in the biosynthesis of dicaffeoyl derivative) have been introduced into said cells of the plant or an ancestor thereof, using genetic engineering, i.e. by human intervention. A heterologous gene may replace an endogenous equivalent gene, i.e. one which normally performs the same or a similar function, or the inserted sequence may be additional to the endogenous gene or other sequence. Nucleic acid heterologous to a plant cell may be non-naturally occurring in cells of that type, variety or species. Thus the heterologous nucleic acid may comprise a coding sequence of or derived from a particular type of plant cell or species or variety of plant, placed within the context of a plant cell of a different type or species or variety of plant. A further possibility is for a nucleic acid sequence to be placed within a cell in which it or a homologue is found naturally, but wherein the nucleic acid sequence is linked and/or adjacent to nucleic acid which does not occur naturally within the cell, or cells of that type or species or variety of plant, such as operably linked to one or more regulatory sequences, such as a promoter sequence, for control of expression. Hence, by means of activation or promoter tagging, i.e. placing expression control sequences functionally adjacent to the target gene, it is also possible to induce expression of the cell's, e.g. plant cell's endogenous gene corresponding to the nucleic acid molecule identified in accordance with present invention; see, e.g., for review Pereira, Transgenic Res. 9 (2000), 245-260 and Dixon and Steele Trends Plant Sci. 4 (1999), 394-400 as well as van der Graaff et al., Plant J. 32 (2002), 819-830. It is to be understood that also these types of transgenic plant are encompassed in the present invention at least as far a change in the phenolic profile, i.e. content of depsides is achieved, either stably or transiently depending on the expression system used.

Genetic modifications of the phenylpropanoid pathway which lead to an increased metabolite flux toward an intermediate and/or of the corresponding desired compound, i.e. depside are, for example, increasing the activity of at least one enzyme of the phenylpropanoid pathway of the wild type, for example by overexpressing genes of the phenylpropanoid pathway identified in accordance with the present invention which encode proteins with this enzymatic activity by switching off negative regulatory mechanisms of metabolic pathways leading to the intermediate or final product, such as, for example, switching off the feedback inhibition or introduction of orthologous genes which are not subject to regulation in the desired organism.

Introduction into the organism of at least one gene to which no orthologous gene exists in the wild type and which bridges the metabolic pathway of the phenylpropanoid pathway of the wild type. For example, this gene may cause, owing to the new gene function, an increased substance flux toward the intermediate or final product where bridging ends.

In addition, or alternatively a target gene, the expression of which has been identified in accordance with the present invention to be down-regulated at a stage of increased depside production, is suppressed, for example via use of antisense or RNAi techniques. Furthermore, inactivation of genes which encode enzymes which compete with the enzymes of the metabolic pathway leading to the desired product is envisaged.

Methods for altering levels in plants of one or more phenolic compounds that are intermediates or final products of the plant phenylpropanoid pathway are described in US patent application No. 2003/150011, the disclosure content of which is incorporated herein by reference and which teaching may be adapted in accordance with the present invention.

Techniques for transforming plant cells with nucleic acids are generally available and can be adapted to the invention by the use of nucleic acid molecules of the present invention. In addition to Berger, Ausubel and Sambrook, useful general references for plant cell cloning, culture and regeneration include Jones (ed) (1995) Plant Gene Transfer and Expression Protocols - Methods in Molecular Biology, Volume 49 Humana Press Towata NJ; Payne et al. (1992) Plant Cell and Tissue Culture in Liquid Systems John Wiley & Sons, Inc. New York, NY (Payne); and Gamborg and Phillips (eds) (1995) Plant Cell, Tissue and Organ Culture; Fundamental Methods Springer Lab Manual, Springer-Verlag (Berlin Heidelberg New York) (Gamborg). A variety of cell culture media are described in Atlas and Parks (eds) The Handbook of Microbiological Media (1993) CRC Press, Boca Raton, FL (Atlas). Additional information for plant cell culture is found in available commercial literature such as the Life Science Research Cell Culture Catalogue (1998) from Sigma-Aldrich, Inc (St Louis, MO) (Sigma-LSRCCC) and, e.g., the Plant Culture Catalogue and supplement (1997) also from Sigma-Aldrich, Inc (St Louis, MO) (Sigma-PCCS). Additional details regarding plant cell culture are found in Croy, (ed.) (1993) Plant Molecular Biology Bios Scientific Publishers, Oxford, U.K.

The DNA constructs of the invention, for example plasmids, cosmids, phage, naked or variously conjugated-DNA polynucleotides (e.g., polylysine-conjugated DNA, peptide-conjugated DNA, liposome-conjugated DNA, etc.), or artificial chromosomes, can be introduced directly into the genomic DNA of the plant cell using techniques such as electroporation and microinjection of plant cell protoplasts, or the DNA constructs can be introduced directly to plant cells using ballistic methods, such as DNA particle bombardment.
Where the sequence is expressed, the sequence is optionally combined with transcriptional and translational initiation regulatory sequences which direct the transcription or translation of the sequence from the exogenous DNA in the intended tissues of the transformed plant.
The present invention also provides methods comprising introduction of a construct of the invention into a plant cell or a microbial cell and/or induction of expression of a construct within such cell, by application of a suitable stimulus, e.g., an effective exogenous inducer.

Microinjection techniques for injecting, e.g., cells, embryos, and protoplasts, are known in the art and well described in the scientific and patent literature. For example, a number of methods are described in Jones (ed) (1995) Plant Gene Transfer and Expression Protocols - Methods in Molecular Biology, Volume 49 Humana Press Towata NJ, as well as in the other references noted herein and available in the literature. For example, the introduction of DNA constructs using polyethylene glycol precipitation is described in Paszkowski et al., EMBO J. 3 (1984), 2717. Electroporation techniques are described in Fromm et al., Proc. Natl. Acad. Sci. USA 82 (1985), 5824. Ballistic transformation techniques are described in Klein et al., Nature 327 (1987), 70-73. Additional details are found in Jones (1995), supra.

Alternatively, and in some cases preferably, Agrobacterium mediated transformation is employed to generate transgenic plants. Agrobacterium-mediated transformation techniques, including disarming and use of binary vectors, are also well described in the scientific literature; see, for example Horsch et al., Science 233 (1984), 496; and Fraley et al., Proc. Natl. Acad. Sci. USA 80 (1984), 4803 and reviewed in Hansen and Chilton, Current Topics in Microbiology 240 (1998), 22; and Das, Subcellular Biochemistry 29, Plant Microbe Interactions (1998), 343-363.

Transformed plant cells which are derived by any of the above transformation techniques can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans et al. (1983) Protoplasts Isolation and Culture, Handbook of Plant Cell Culture pp. 124-176, Macmillian Publishing Company, New York; and Binding (1985) Regeneration of Plants, Plant Protoplasts pp. 21-73, CRC Press, Boca Raton. Regeneration can also be obtained from plant callus, explants, somatic embryos (Dandekar et al., J. Tissue Cult. Meth. 12 (1989), 145; McGranahan et al., Plant Cell Rep. 8 (1990), 512), organs, or parts thereof. Such regeneration techniques are described generally in Klee et al., Ann. Rev. of Plant Phys. 38 (1987), 467486. Additional details are found in Payne (1992) and Jones (1995), both supra. These methods are adapted to the invention to produce transgenic plants bearing genes isolated according to the methods of the invention.

Preferred plants for the transformation and expression of genes and other nucleic acids identified and cloned according to the present invention include agronomically and horticulturally important species. Such species include, but are not restricted to members of the families: *Graminae* (including corn, rye, triticale, barley, millet, rice, wheat, oats, etc.); *Leguminosae* (including pea, beans, lentil, peanut, yam bean, cowpeas, velvet beans, soybean, clover, alfalfa, lupine, vetch, lotus, sweet clover, wisteria, and sweetpea); *Compositae* (the largest family of vascular plants, including at least 1,000 genera, including important commercial crops such as sunflower) and *Rosaciae* (including raspberry, apricot, almond, peach, rose, etc.), as well as nut plants (including, walnut, pecan, hazelnut, etc.), and forest trees (including Pinus, Quercus, Pseutotsuga, Sequoia, Populus, etc.). Additionally, preferred targets for modification with the nucleic acids of the invention, as well as those specified above, plants from the *genera : Agrostis, Allium, Antirrhinum, Apium, Arachis, Asparagus, Atropa, Avena* (e.g., oats), *Bambusa, Brassica, Bromus, Browaalia, Camellia, Cannabis, Capsicum, Cicer, Chenopodium, Chichorium, Citrus, Coffea, Coix, Cucufrzis, Curcubita, Cynodon, Dactylis, Datura, Daucus, Digitalis, Dioscorea, Elaeis, Eleusine, Festuca, Fragaria, Geranium, Glycine, Helianthus, Heterocallis, Hevea, Hordeum* (e. g., barley), *Hyoscyamus, Ipomoea, Lactuca, Lens, Lilium, Linus, Lolium, Lotus, Lycopersicon, Majorana, Malus, Maragifera, Manihot, Medicago, Nemesia, Nicotiana, Onobrychis, Oryza* (e.g., rice), *Panicum, Pelargonium, Pennisetum* (e.g., millet), *Petunia, Pisum, Phaseolus, Phleum, Poa, Prunus, Ranunculus, Raphanus, Ribes, Ricinus, Rubus, Saccharum, Salpiglossis, Secale* (e.g., rye), *Senecio, Setaria, Sinapis, Solanum, Sorghum, Stenotaphrum, Tlaeobro za, Trifolium, Trigonella, Triticum* (e.g., wheat), *Vicia, Vigna, Vitis, Zea* (e.g., corn), and the *Olyreae,* the *Pharoideae* and many others. As noted, plants in the family *Asteraceae* are a particularly preferred target plants for transformation with cloned sequences by the methods of the invention.

Common crop plants which are targets of the present invention include corn, rice, triticale, rye, cotton, soybean, sorghum, wheat, oats, barley, millet, sunflower, canola, peas, beans, lentils, peanuts, yam beans, cowpeas, velvet beans, clover, alfalfa, lupine, vetch, lotus, sweet clover, wisteria, sweetpea and nut plants (e.g., walnut, pecan, etc.).

In a preferred embodiment, said transgenic plant is sunflower *(Helianthus annuus L.).* Stable transformation of sunflower using a non-meristematic regeneration protocol and green fluorescent protein as a vital marker is described for example in Muller et al., Transgenic Res. 10 (2001), 435-444.

Cells that express reporter genes in transient assays may not give rise to cells where the transformed DNA becomes stably integrated into the host cell genome. Selection of cells that express various marker genes, however, does give rise to cells in which the transformed DNA is stably integrated into the host cell genome. Herein, "selection" means conditions where only cells into which the DNA construct has been delivered will grow and cells in which the DNA construct has not been delivered will not grow. For example, cells stably expressing an introduced neomycin phosphotransferase gene are selected by growth in the drug G418. Cells stably expressing an introduced herbicide resistance gene are selected by growth in the presence of the herbicide. Shoots or plantlets growing in the presence of the drug or herbicide are presumptively transformed. Confirmation of stable integration of the transformed genes into the genome of the host may later be accomplished by, for example, herbicide treatment of the resulting plants. In addition, later molecular detection of the introduced DNA in the isolated genomic DNA of the plant cells, for example using Southern blotting/hybridization or polymerase chain reaction, may be used to confirm integration of the introduced genes into the genome of the host.

Transformed plant host cells are used to regenerate transgenic plants. In plants every cell is capable of regenerating into a mature plant and, in addition, contributing to the germ line such that subsequent generations of the plant will contain the transgene. Growth of transformed plant cells and regeneration of such cells into mature plants is routine among those skilled in the art.

The transgenic plants are then grown and pollinated with either the same transformed strain or with different strains, and the resulting hybrid, having the desired phenotypic characteristic, is identified. Two or more generations may be grown to ensure that the desired phenotypic characteristic is stably maintained and inherited and then seeds harvested. Transformed progeny obtained by this method may be distinguished from non-transformed progeny by the presence of the introduced transgene(s) and/or accompanying DNA in the genome of the plant. Transformed plants also may be distinguished from non-transformed plants by a change in phenotype. For example, transformed plants may be distinguished from non-transformed plants by the presence of the introduced recombinant DNA in tissues or cells where it normally is not present or by an increase or decrease in the amount of the encoded enzyme in cells where it normally is present.

For some embodiments, it may be appropriate to introduce into the plant two or more different nucleic acids, at least one of which has been identified and provided in accordance with the present invention. In addition thereto, genes that have been described in the prior art or otherwise provided may be employed in order to arm the subject plant with a complete set of enzymes for the production of the desired depsides; see also supra.

The present invention also encompasses plant cells which are transiently transfected with an RNA or DNA molecule which encodes an enzyme involved in the biosynthesis and/or accumulation of depsides. Such cells are useful for producing large amounts of desirable phenolic compounds that are intermediates or final products of the phenylpropanoid pathway.

Hence, the present invention also relates to transgenic plants and plant cells obtainable by the above-described method of the invention, preferably wherein the presence of said recombinant cDNA identified in accordance with the present invention leads to a change in a phenotypic trait as mentioned above, preferably to an increase of the content of depsides.

Preferably, increasing the depside content means for the purposes of the present invention the artificially acquired ability of increased biosynthesis of these compounds in the plant in comparison with the plant which has not been modified by genetic engineering over at least one plant generation.

Such transgenic plants, their propagation material, and their plant cells, plant tissues or plant parts are a further subject matter of the present invention. As mentioned, plants for the purposes of the invention are preferably members of the plant family *Asteraceae, Boraginaceae, Solanaceae, Lamiaceae,* or *Apiaceae,* most preferably selected from the group consisting of artichoke *(Cynara),* chrysanthemum *(Chrysanthemum),* cornflower *(Centaurea),* coneflower *(Echinacea ssp.*)*,* lettuce *(Lactuca ssp.),* sagebrush, motherwort, mugwort, wormwood, jerusalem artichoke *(Artemisia ssp.),* and annual and perennial sunflower *(Helianthus ssp.),* and particularly preferred the plant is sunflower.

The genetically modified organisms, in particular plants, can be used as described above for the production of depsides. Genetically modified plants according to the invention which can be consumed by humans and animals and which have an increased content of depsides may also be used as foodstuffs or feedstuffs, for example directly or after processing in a manner known per se.

Furthermore, the present invention relates to harvestable parts of such plants comprising transgenic plant cells, for example progeny, seed or fruits.

In a further embodiment, the present invention relates to a process for the production of depsides, which process comprises
(a) subjecting a substrate comprising phenolic carboxylic acids to a host cell of the present invention or a culture medium thereof, a polypeptide of the present invention or to an extract of a plant of the present invention or of a seed or fruits thereof, and
(b) isolating the depsides so produced.
As substrate extracts of appropriate plants may be used and/or isolated phenylpropanic acids like cinnamic acid, ferulic acid, p-coumaric acid, caffeic acid, 5-hydroxyferulic acid, and/or sinapic acid.

Phenolic compounds which can be prepared in accordance with the method of the present invention include depsides, in particular dicaffeoyl derivatives including 1-, 5-O-mono- and 3,4-0-bis-substituted derivatives of quinic acids (see, e.g., US patent No. 5,395,950) and 3- or 4-mono-, 1,3- or 1,4- or even 3,4-disubstituted derivatives of quinic acids (see, e.g., US patent No. 5,401,858). Most preferably, dicaffeoyl containing compounds are prepared such as those described above as well as derivatives such as dicaffeoyl-L-tartaric acid (L-chicoric acid, L-DTCA or L-CCA), dicaffeoyl-D-tartaric acid (D-chicoric acid, D-DCTA), dicaffeoyl-mesotartaric acid (meso-chicoric acid, meso-DCTA). Further analogs of chicoric acid such as acetylated compounds are described in international application WO00/63152.

Further compounds which may be produced in accordance with any one of the above described methods of the present invention comprise, for example hydroxyphenyl derivatives with HIV integrase inhibitory properties as described in US patent No. 6,362,165. Likewise, it may be possible to produce with the in vitro or in vivo methods of the present invention dicaffeoylquinic acid derivatives such as described in international application WO98/09599. These compounds have been described to be useful for treating Hepatitis B and diseases associated with retroviral infection (such as HIV).

With one major limiting factor in anti-HIV therapy being the costs of the antiviral agents, especially for those that have to be synthesized by complex organic chemistry, the methods of the present invention allow a considerable reduction of costs which could substantially increase the number of patients who could be on combination therapy regimens. Hence, the present invention also relates to a process of manufacturing an anti-viral composition comprising the steps of any one of the above described methods, and optionally formulating the depside with a pharmaceutically acceptable carrier. Preferably, said anti-viral composition is an anti-HIV medicament.

For this purpose, each compound so produced can be pursued using biological characterization with living cells, including determination of LD50 by cell toxicity assay, determination of ED50 by anti-HIV assay, and assessment of selectivity against integrase, biochemical characterization, i.e. determination of IC50 with the disintegration assay (Chow, Science 255 (1992), 723-726), and determination of pharmacological compatibility of anti-HIV activity when used in combination with members of the established classes of anti-HIV therapeutics, i.e. determination of capacity to act synergistically. Such course of assessment is presented for example in international application WO99/48371.

In a preferred embodiment, the composition according to the invention is useful in therapeutic applications or in prophylactic applications. Of particular interest is, for example, the possible use for vaccination by oral application. Such methods of treatment, their dosage levels and requirements may be selected by those of ordinary skill in the art from available methods and techniques. For example a compound of this invention may be combined with a pharmaceutically acceptable adjuvant for administration to a virally infected patient in a pharmaceutically acceptable manner and in an amount effective to lessen the severity of the viral infection. Also, a compound of this invention may be combined with pharmaceutically acceptable adjuvants conventionally employed in vaccines and administered in prophylactically effective amounts to protect individuals over an extended period of time against viral infections, such as HIV infection. The compounds of this invention may be administered to a healthy or HIV-infected patient either as a single agent or in combination with other antiviral agents which interfere with the replication cycle of HIV. Although this invention focuses on the use of the extracts and compounds disclosed herein for preventing and treating HIV infection, the compounds of this invention can also be used as inhibitory agents for other viruses that depend on similar integrases for obligatory events in their life cycle. These viruses include, but are not limited to, other diseases caused by retroviruses, such as simian immunodeficiency viruses, HTLV-I and HTLV-II.

However, as mentioned above, the depsides prepared according to the present invention as well as extracts from transgenic organisms, for example transgenic plants or parts thereof are suitable for use in the treatment of inflammatory disease and tumors as well. Thus, the present invention also relates to pharmaceutical compositions comprising any of the extracts or compounds, i.e. depsides produced in accordance with the present invention, and optionally any pharmaceutically acceptable carrier, adjuvant or vehicle for the treatment of inflammatory diseases, for example allergic diseases or for the treatment of tumors.

Pharmaceutical compositions of this invention comprise any of the extracts or compounds, i.e. depsides produced in accordance with the present invention, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethyleneglycol, sodium carboxymethylcellulose, polyacrylates, waxes, polyethylene-polyoxypropylene-block polymers, polyethylene glycol and wool fat.

The pharmaceutical compositions of this invention may be administered orally, parenterally by inhalation spray, topically, rectally, nasally, buccally, vaginally or via an implanted reservoir. Preferred are oral administration or administration by injection. The pharmaceutical compositions of this invention may contain any conventional non-toxic pharmaceutically acceptable carriers, adjuvants or vehicles. The term "parenteral" as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solutions. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv. or a similar alcohol.

The appropriate concentration of the therapeutic agent might be dependent on the particular agent. The therapeutically effective dose has to be compared with the toxic concentrations; the clearance rate as well as the metabolic products play a role as do the solubility and the formulation. Therapeutic efficacy and toxicity of compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. Specific dosage and treatment regimen for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician.

The compounds of this invention are also useful as commercial reagents which effectively bind to integrases, particularly HIV integrase. As commercial reagent, the compounds of this invention, and their derivatives, may be used to block integration of a target DNA molecule by integrase, or may be derivatized to bind to a stable resin as a tethered substrate for affinity chromatography applications. These and other uses which characterize commercial integrase inhibitors will be evident to those of ordinary skill in the art.

These and other embodiments are disclosed and encompassed by the description and Examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc.) are hereby expressly incorporated by reference; however, there is no admission that any document cited is indeed prior art as to the present invention.

The above disclosure generally describes the present invention. A more complete understanding can be obtained by reference to the following specific Examples which are provided herein for purposes of illustration only and are not intended to limit the scope of the invention.

### EXAMPLE

The example which follow further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. ed by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art.
Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11, (2001), 98-107.

### Example: Differential induction of dicaffeoylquinic and dicaffeoyltartaric acids in Asteraceae

The induction of Dicaffeoylquinic- and Dicaffeoyltartaric- acids (DCQA and DCTA) in *Asteraceae* by *Sclerotinia sclerotiorum* comprises several subsequent steps which are shown in Figure 1 and exemplified for the two perennial *Helianthus maximiliani* Accessions AC M and AC 7.

### Origin of the biological material

*H maximiliani* AC M can be obtained from the Botanical Garden Lisbon, Portugal [Jardim Botânico da Faculdade de Ciência da Universidade Lisbon, P-1250 Lisbon]. *H. maximiliani* AC 7 can be obtained from the North Central Regional Plant Introduction Station, Iowa State University; USDA-ARS; Ames, IA 50011-1170, USA. *Sclerotinia sclerotiorum* isolates can be obtained from DSMZ - Deutsche Sammlung von Mikroorganismen und Zellkulturen, Mascheroder Weg 1B, D-38124 Braunschweig, Germany.

### Maintenance and propagation of Sclerotinia sclerotiorum isolates

Cultures of *Sclerotinia sclerotiorum* isolates can be sterilized, maintained and propagated according to Grezes-Besset et al., Plant Breeding 112 (1994), 215 - 222.

### 1. Sterilization of Helianthus maximiliani rhizomes

Initial explants for plant tissue culture consisting of sterile young rhizomes collected in autumn from field grown perennial *Helianthus maximiliani* Accessions. Sterilization of the rhizomes can be performed by the procedure described in Imhoff et al., Appl. Bot. 70 (1996), 137-139.

### 2. In vitro propagation of Helianthus maximiliani species

Sterile initial plants obtained from emerging rhizomes are micropropagated according to Imhoff et al., (1996) supra, with slight modifications. Abscised terminal buds and internodal segments are placed on Murashige and Skoog agar media (MS-Media) (Murashige & Skoog, Physiol. Plant. 15 (1962), 473-497) containing 3% sucrose and 0.6g L⁻¹ MES (2[-morpholino] ethanesulfonic acid) and adjusted to pH 5.7. Plants are kept by a photoperiod of 14h light and 10h dark, a temperature of 27°C and under a relative humidity of approx. 70% rH.

### 3. Callus induction of Helianthus maximiliani

Callus cultures of wild sunflowers (AC M and AC 7) are initiated from sterile young sliced leaf segments placed on a basal agar medium containing MS-Media (Murashige & Skoog, 1962) with 3% sucrose, 100mg L⁻¹ inositol, Gamborgs B5 vitamines according to Gamborg et al. (Gamborg et al., Exp. Cell Res. 50 (1968), 151-158), pH 5.7 and supplemented either with 0.2mg L⁻¹ NAA (Napthylacetic Acid) and BAP (Benzylaminopurine) for AC M or 2mg L⁻¹ NAA and BAP for AC 7. Cultures are incubated in the dark at a temperature of 25°C.

### 4. Cocultivation with Sclerotinia sclerotiorum - DCQA (DCTA) induction of callus cultures

Four week old callus cultures are used for the induction of DCQA's. Callus should be equal or greater than 1 cm in diameter. Mycelium plugs - 1 cm in diameter - from *Sclerotinia sclerotiorum* cultures are placed into the middle of the callus culture. Agar plates are completely sealed with Parafilm and incubated in the dark at a temperature of 25°C. Two days after initial cocultivation of *H. maximiliani* callus with S. *sclerotiorum,* when the mycelium has completely overgrown the calli, the plant tissue culture can be subsequently transferred to DCQA extraction.

### 5. Extraction and preparation of DCQA (DCTA) enriched fractions

Cells of *H maximiliani* callus are ground on ice in a 4°C cold solution of methanol and water (1:1 v/v) and subsequently centrifugated for 10min at 15.000rpm. After centrifugation the supernatant is carefully separated from cell fragments and kept on ice until further fractionating. Extraction of phenolic components can be performed by solid phase extraction (SPE, ENVI-Chrom P Column, Supelco, Bellefonte, P.A., USA) in which phenolic compounds with altering physicochemical behaviour are separated according to their adsorption to the SPE matrix. For the preparation of highly DCQA enriched fractions a solution gradient within the range of 0 and 100% of acetonitril and methanol:water (1:1 v/v) is applied to the cell raw extract (supernatant). DCQA enriched fractions can be obtained within a range between 35 - 50 : 65 - 50 acetonitril : methanol/water (v/v). Therefore the SPE column is preconditioned with 2ml acetonitril followed by 2mL water. Subsequently 2mL of the clear cell supernatant is added to the SPE column and rinsed by means of vacuum pressure (Visiprep Solid Phase Extraction Vacuum Manifold, Supelco, USA). In order to elute DCQA compounds the tube is rinsed with 2 x 0.5mL of the appropriate solvent (acetonitril : methanol/water - see above). Aliquots of the elutes should be carefully lyophilized (-20°C, - 0.47bar to +20°C, normal pressure within 20h) prior to chromatographic separation.

### 6. Chromatographic separation and identification of DCQA's and DCTA's

Lyophilized probes are dissolved in 20µL methanol/water prior to the chromatographic separation. 5µL aliquots of the eluates and 2µL standards of caffeic- and chlorogenic acid (1mg of each substance dissolved in 10mL methanol) are applied as 8mm bands on HPTLC plates (HPTLC silica gel 60, 20x10cm; Merck, Darmstadt, Germany) at least 2mm apart and 8mm from lower edge of the plate. Chromatographic separation can be performed by a mobile phase consisting of ethyl acetate : ethylmethyl ketone : formic acid : water (15:9:3:3) and can be conducted in a horizontal TLC chamber (20x10 Twin Trough Chamber, CAMAG, Sonnenmattstr. 11, CH-4132 Muttenz). Developing distance should be 60-70mm from the lower edge of the plate and HPTLC chromatograms are dried for 5min in a cold air stream. Identification of DCQA's and DCTA's can be conducted by R_{f} values and colour of the spots before and after derivatization with Neu's reagent (1g diphenylborinic acid aminoethylester dissolved in 200mL methanol (Neu, Naturwissenschaften 43 (1956), 83) and examined under 366nm UV-light.
Under the above mentioned mobile phase and according to CAMAG 2003, (CAMAG Application Notes Nr. 11/03 [F-24A]: HPTLC Identification of *Echinacea: E. purpurea, E. angustifolia, E. pallida -* Phenolics; CAMAG AG, CH-4132 Muttenz) and Bauer et al. 1986 & 1988 (Bauer et al., *Dtsch. Apoth. Ztg.* 126 (1986), 1065-1070; Bauer et al., Planta Medica 54 (1988), 426-430) the following chromatographic characteristics (R_{f} values and colours under visible and UV light) for monocoffeoyl acids (MCQA; chlorogenic acids) DCQA's and DCTA's can be obtained; see Table 1 below:

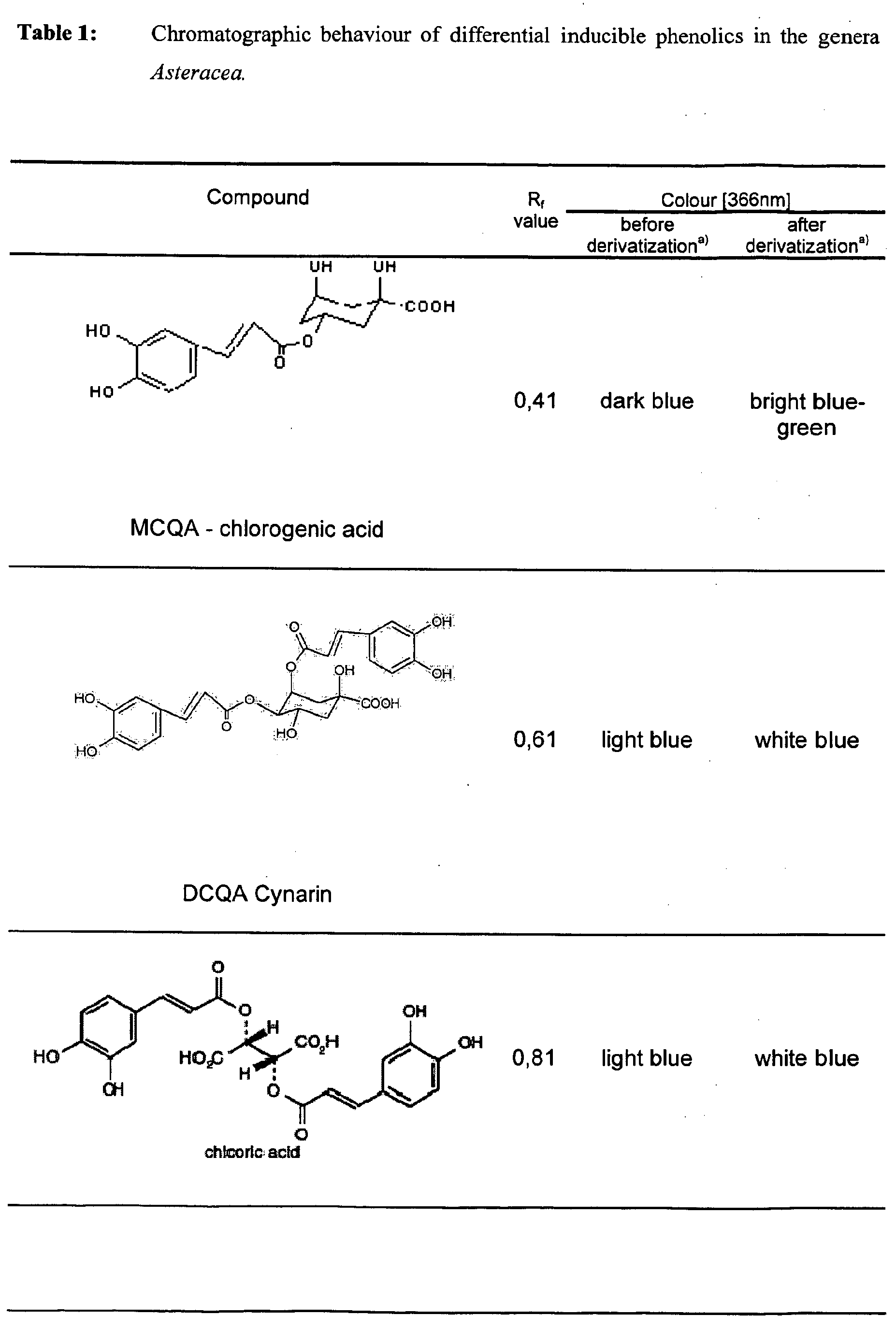

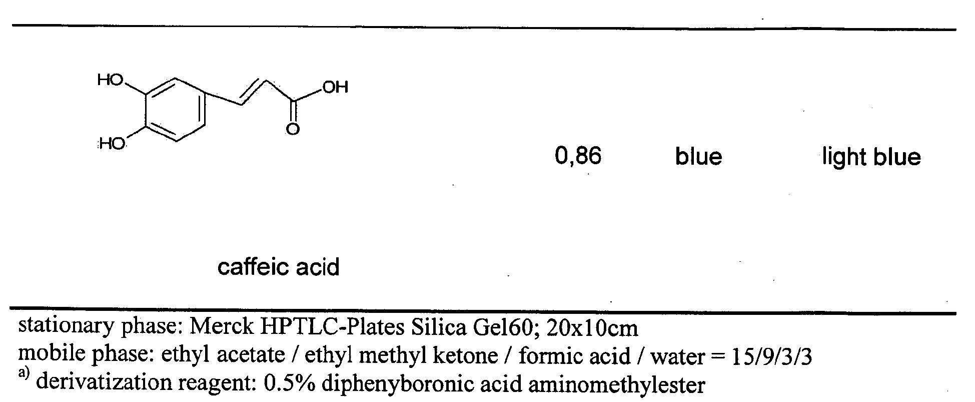

### 7. Identification of DCQAs performing LC/ESI-MS

Application samples for LC/ESI-MS measurements are callus cultures *Helianthus maximiliani* AC 7 grounded with methanol/water (1:1, v:v) after DCQA induction by *Sclerotinia sclerotiorum* as described under 4.
The analyses were performed using an Agilent 1100 HPLC (Agilent Technologies, Palo Alto, CA, USA) interfaced to an Applied Biosystems API 2000 triple-quadrupole mass spectrometer (Applied Biosystems, Foster City, CA, USA). The mass spectrometer was operated in turbo ion spray negative ion mode using multiple reaction monitoring (MRM). MS instrument tuning was conducted for each analyte (1,5 di-caffeoylquinic acid, caffeic acid and chlorogenic acid) by direct-infusion of a ~ 1 µg/mL standard solution (50% water / 50% methanol) at a flow rate of 10 µL/min. The analyte was initially tuned for the parent ion and then subsequently tuned for the product ions. Typically, the following tune parameters were used: drying gas temperature 350 °C; ion spray voltage -3500 V; curtain gas pressure 25 psi; nebuliser gas pressure 40 psi; drying gas pressure 70 psi. The settings for ion focusing and fragmentation were optimized for each analyte individually. For qualitative and quantitative purposes, the instrument was operated in MRM mode, monitoring the transition:

| | |
|---|---|
| 1,5 di-caffeoylquinic acid (M-H): | m/z 515 → 191 m/z |
| | m/z515 →179m/z |
| | m/z515 →135m/z |
| | m/z 515 → 353 m/z |
| | |
| caffeic acid (M-H): | m/z 179 → 135 m/z |
| | m/z 179 → 89 m/z |
| | |
| chlorogenic acid (M-H): | m/z 353 → 191 m/z |
| | m/z 353 → 85 m/z |

The Agilent 1100 HPLC column was a Synergi Polar-RP 2,0 x 150 mm, 4 µm particle size (Phenomenex, Torrance, USA), maintained at a temperature of 30 °C. The injection volume was 10 µL. The mobile phase A was 0,1% v/v formic acid in water and the mobile phase B was methanol. The following gradient was used; see Table 2 below:

**Table 2: HPLC-gradient for LC/ESI-MS**

| | | | | | | |
|---|---|---|---|---|---|---|
| Time [min] | 0 | 1 | 15 | 20 | 20,5 | 35 |
| Solvent A[%], | 80 | 80 | 25 | 25 | 80 | 80 |
| Solvent B [%] | 20 | 20 | 75 | 75 | 20 | 20 |
| Flow[mL min⁻¹] | 0,2 | 0,2 | 0,2 | 0,2 | 0,2 | 0,2 |

Fortification solutions of Cynarin (1,5 di-caffeoylquinic acid), caffeic acid and chlorogenic acid, respectively, are applied for identification and quantification of di-caffeoyl-quinic acids.

### 8. Characterization of different isomeric forms of DCQAs

Samples are prepared as described under 7. DCQA's are characterized performing methods of identification as described in Clifford et al., J. Agric. Food Chem. 53 (2005), 3821-3832, applying slight modifications. Eluents used for separation are water/acetonitrile/acetic acid (980/20/5, v/v/v, (A)) and acetonitrile/acetic acid (1000/5, v/v, (B)). HPLC is equipped with a LUNA Phenyl-Hexyl column 150 x 2,0 mm, 3 µm particle size (Phenomenex, Torrance, USA) and 100 µl aliquot of callus extract is injected on the column. The gradient profile (0.3 ml min⁻¹) is programmed stepwise from 4 % B to 33 % B in 90 min, followed by an increase of solvent B to 100 % in 5 min, then 5 min isocratic, and finally a decrease of solvent B to 4 % in 5 min; see Table 3, below. Mass spectrometry was performed as described under 7.

**Table 3: HPLC-gradient for LC/ESI-MS (according to Clifford et al., supra)**

| | | | | | |
|---|---|---|---|---|---|
| Time [min] | 0 | 80 | 95 | 100 | 105 |
| Solvent A [%] | 96 | 67 | 0 | 0 | 96 |
| Solvent B [%] | 4 | 33 | 100 | 100 | 4 |
| Flow [mL min⁻¹] | 0,3 | 0,3 | 0,3 | 0,3 | 0,3 |

Different isomeric forms of the DQCA's occuring in induced stem callus cultures of *H maximiliani* were separated and identified according to their esterification of caffeoyl-residues, as shown in Table 4, below.

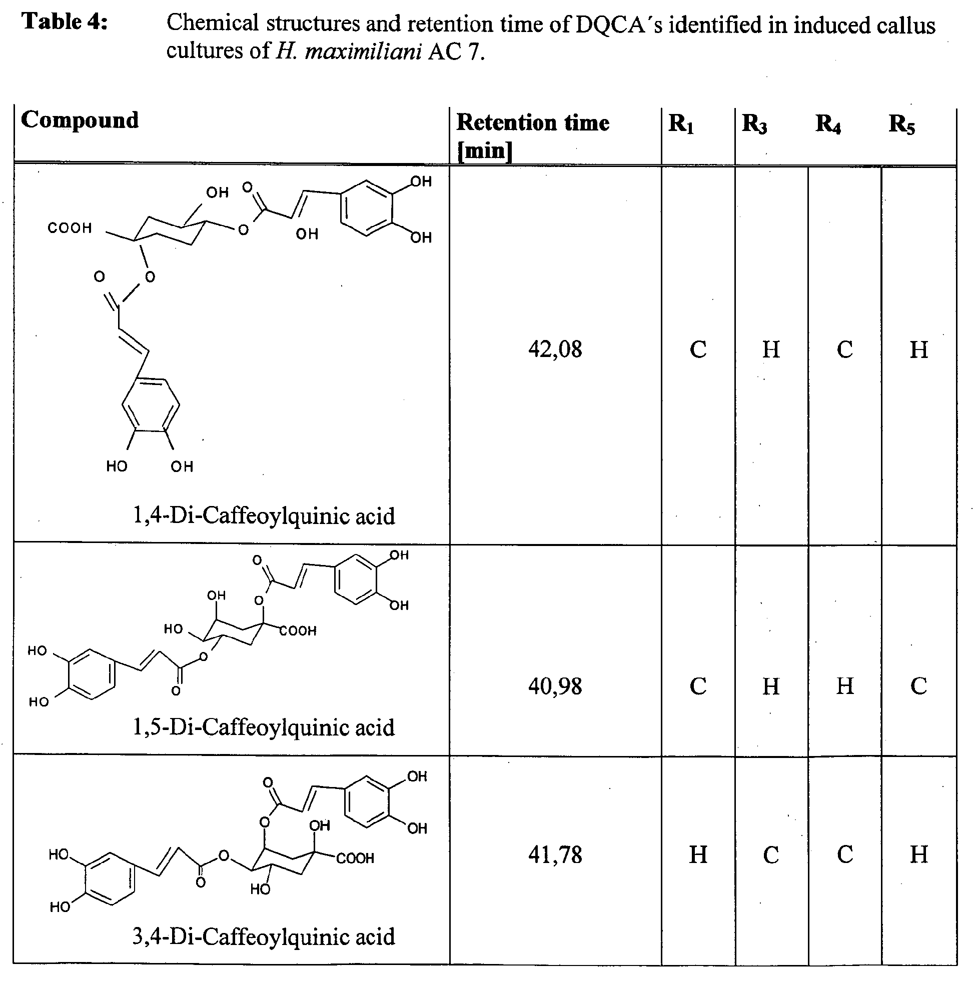

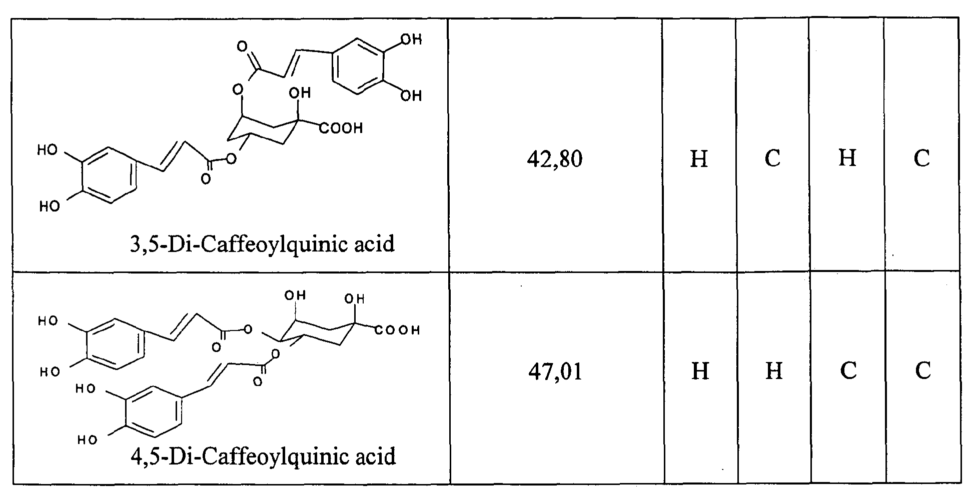

### 9. Isolation and analysis of differentially expressed gene fragments

The method of DDRT-PCR (Differential Display Reverse Transcriptase-PCR) makes it possible to compare mRNA expressed of two or more different cell populations and was first described by Liang and Pardee, Science 257 (1992), 967-971. Figure 7 shows the different maintained steps adapted for DDRT-PCR analysis.

### 10. Isolation and cultivation of mesophyll protoplasts

Sterile leaves of *H. maximiliani* AC 7 are cut into pieces and digested in the dark for 16 h in a mixture of enzymes containing 0.15 % cellulase, 0.05 % pectolyase, 0.75 % macerozyme, 0.005 % driselase and 1 % bovine serum albumin dissolved in a salt solution for mesophyll protoplast isolation at pH 5.6 containing KCl (340 mM), CaCl₂ x 2H₂O (0,14 mM) and MES (3 mM) according to Henn et al., Plant Cell Reports 18 (1998), 288-291. For isolation of protoplasts, the enzyme-protoplast mixture is filtered through 100 and 50 µm sieves and centrifugated at room temperature for 6 min at 50 g. The pellet is resuspended with 6 ml sucrose solution at pH 5.6, containing sucrose (0.5 M), CaCl₂ x 2H₂O (0.14 mM) and MES (3 mM) and covered with 1 ml salt solution. Again, the solution is centrifugated for 6 min at 50 g. Protoplasts are taken from the interphase and cultivated in mKM-medium (Table 5) for 36 h with a density of 2.5 x 10⁴ ml⁻¹ until the cell wall is reestablished.

**Table 5: Components of mKM-medium for cultivation of mesophyll protoplasts according to Wingender et al., Plant Cell Rep. 15 (1996), 742-745.**

| **Stock No.** | **Compound** | **Concentrati on [mg/l]** | **Stock No.** | **Compound** | **Concentrati on [mg/l]** |
|---|---|---|---|---|---|
| **Ia:** | CaCl₂*2H₂O | 1470 | **IV:** | Ascorbic Acid | 2,0 |
| | KNO₃ | 760 | | Ca-Panthotenate | 1,0 |
| | NH₄NO₃ | 400 | | Cholinechloride | 1,0 |
| | | | | Nicotineamide | 1,0 |
| **Ib:** | MgSO₄*7H₂O | 985 | | Pyridoxal - HCl | 1,0 |
| | KH2PO4 | 68 | | Thiamine | 1,0 |
| | **10ml Ia/b I**^{**-1**} | | | **5ml IV I**^{**-1**} | |
| **Ic:** | FeNaEDTA | 38,5 | V: | Na-Pyruvate | 20 |
| | **10ml Ic I**^{**-1**} | | | Fumaric Acid | 40 |
| | | | | Citric Acid | 40 |
| **IIa:** | CoCl₂ *6H₂O | 0,024 | | Malic Acid | 40 |
| | CuSO₄ | 0,0158 | | | |
| | Na₂MoO₄*2H₂O | 0,120 | | adjusted with NH₄OH to pH 5,5 | |
| | **100µl IIa I**^{**-1**} | | | **10ml V l**^{**-1**} | |
| **IIb:** | MnSO₄*4H₂O | 11,15 | **VI:** | Aminobenzoic Acid | 0,02 |
| | H₃BO₃ | 3,1 | | Biotine | 0,01 |
| | ZnSO₄*7H₂O | 1,44 | | Vitamine A | 0,01 |
| | KJ | 0,83 | | pre dissolved in EtOH | |
| | **10ml IIb l**^{**-1**} | | | **100µl VI l**^{**-1**} | |
| **III:** | Cellobiose | 250 | VIa: | Vitamine D₃ | 0,01 |
| | Fructose | 250 | | Vitamine B₁₂ | 0,02 |
| | Inositol | 250 | | 200µl VIa l⁻¹ | |
| | Mannose | 250 | | | |
| | Rhamnose | 250 | VII: | Caseinhydrolysate | 250 |
| | Ribose | 250 | | 10ml VII l⁻¹ | |
| | Sucrose | 250 | | | |
| | Sorbitole | 250 | VIIa: | Riboflavine | 0,2 |
| | Mannitole | 250 | | 4ml VIIa l⁻¹ | |
| | Xylose | 250 | | | |
| | **10ml III I**^{**-1**} | | **VIIb:** | Folic Acid | 0,4 |
| | | | | **4ml VIIb I**^{**-1**} | |

### 10. Cocultivation with S. sclerotiorum - Induction of protoplast cultures

Two week old mycelia of S. *sclerotiorum* is scratched off the agar plate and mixed with sterile mKM-medium. An aliquot of 50 µl of the fungus-mKM-suspension is carefully mixed with 500 µl of mKM-medium containing intact protoplasts (2.5 x 10⁴ ml⁻¹). A time series of 5 variants (0, 10, 30, 60 min and 24 h after treatment with S. *sclerotiorum)* is applied for differential display analysis.

### 11. Analysis of differentially expressed gene fragments

After treatment with S. *sclerotiorum,* suspensions are lysed using a Guanidine-Isothiocyanat-buffer 1:1 (OL1, QIAGEN GmbH, Hilden). Cells are subsequently disrupted using QIAshredder (QIAGEN GmbH, Hilden). Isolation and purification of mRNA is accomplished by Oligotex Direct mRNA Micro Kit (QIAGEN GmbH, Hilden) following conditions recommended by the manufacturer. Total mRNA is reverse transcribed in a final volume of 20 µl using 1^{st} strand cDNA Synthesis Kit for RT-PCR (AMV, Roche) and fluorescence-marked Oligo-dT-N-Primer (T₁₇A-mod; SEQ ID NO: 2, T₁₇C-mod; SEQ ID NO: 3 and T₁₇G-mod; SEQ ID NO: 4, 13.3 µM):

Oligo-dT17-A 5′-FAM-CGG GCC TCT TCG CTA TTT TTT TTT TTT TTT TTA-3′
(SEQ ID NO: 2)

Oligo-dT17-C	5′-FAM-CGG GCC TCT TCG CTA TTT TTT TTT TTT TTT TTC-3′
(SEQ ID NO: 3)

Oligo-dT17-G 5′-FAM-CGG GCC TCT TCG CTA TTT TTT TTT TTT TTT TTG-3′
(SEQ ID NO: 4)

RT-PCR reaction was accomplished starting with an annealing at 25 °C for 10 min., followed by the reverse transcription at 42 °C for 60 min, and finally ending at 99 °C for 5 min.
Random-PCR is performed using 0.5 µl of cDNA as templates, a fluorescence marked reverse primer (Oligo-dT-mod, 13.3 µM) and a modified Oligo-10 forward primer (Random-Primer, BO1-B07, 10 µM, Roth), 5u/µl recombinant Taq DNA polymerase (Fermentas Life Sciences), KCl buffer (Fermentas Life Sciences), MgCl₂ (25 mM, Fermentas Life Sciences), and dNTP's (10 mM) and performed as follows: 1 cycle at 92°C for 5 min; 10 cycles at 92°C for 30 s, 33°C for 60 s, and 72°C for 60 s; 30 cycles at 95°C for 30 s, 64.8°C for 60 s, and 72°C for 60 s; followed by a 10-min extension at 72°C; and hold at 4°C.
Differential display fragment analysis is performed using CEQ™ DTCS-Quick Start Kit (Beckman-Coulter, USA) employing 40 µl SLS (Sequencing loading solution), 0.5 standard (600 bp) and 1 µl template. Separation is performed by capillary gel electrophoresis (CEQ™ 8000, Beckman-Coulter, USA) with an injection at 2.0 kV for 30 s and a separation procedure at 4.8 kV for 60 min.

For sequencing of differentially expressed cDNAs, PCR products are separated on a 3.5 % agarose gel and interesting bands are excised from the gel and purified using a Gel Extraction Kit (Qiagen, Hilden). Reamplification of cDNAs is carried out in a 20 µl-PCR containing 1 µl of cDNA eluate, forward and reverse sequencing primer (M13; SEQ ID NO: 5: 5'CGG GCC TCT TCG CTA 3' and SEQ ID NO: 6: 5'GCC CGG AGA AGC GAT 3'; 12 µM), 5u/µl recombinant Taq DNA polymerase (Fermentas Life Sciences), KCl buffer (Fermentas Life Sciences), MgCl₂ (25 mM, Fermentas Life Sciences), and dNTP's (10 mM) and performed as follows: 1 cycle at 95°C for 5 min; 35 cycles (95°C for 30 s, 64.8°C for 2.5 min, and 72°C for 30 s); followed by a 10-min extension at 72°C; and hold at 4°C. Purity check of reamplified fragments is accomplished by separation using capillary gel electrophoresis as described above. Reamplified cDNAs are sequenced with application of M13-primers (SEQ ID NOs: 5 and 6).

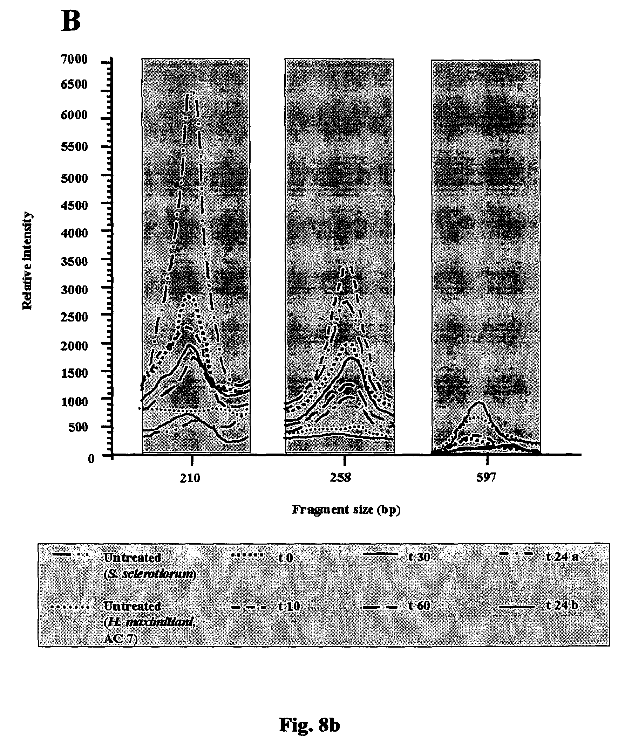

Results of DDRT-RCR analysis are shown in Figure 8 and nucleotide sequence maintained by fragment analysis of differentially expressed mRNA of *H. maximiliani* Schrad is given by SEQ ID NO: 1.

## Claims

1. A method of increasing the content of depsides, preferably dicaffeoylquinic acids (DCQAs) and/or dicaffeoyltartaric acids (DCTAs) in a plant, or corresponding tissue or cell thereof, said method comprising treating said plant, tissue or cell with a biotic and/or abiotic stimuli.

2. The method of clam 1, wherein said biotic stimuli is a plant pathogen, preferably *Sclerotinia* or an elicitor derived thereof.

3. Use of a plant, tissue or cell as defined and treated in claim 1 or 2, with an increased content of depsides, or of products prepared with these plants, tissue or cells such as juice, teas, extracts, fermentation products, and fermentation residues for the preparation of a curative composition, health-promoting composition or tonic, or for a cosmetic or for isolating depsides from said extract of a plant, tissue or cell.

4. A method of identifying and obtaining a nucleic acid molecules correlating with or involved in the biosynthesis or accumulation of depsides in a plant, said method comprising
(a) providing a nucleic acid sample of plant, tissue or cell as defined in claim 1 or 2, said plant, tissue or cell having been treated with a plant pathogen such as *Sclerotinia* or an elicitor derived thereof;
(b) identifying, and optionally isolating a nucleic acid molecule which is differentially expressed in said nucleic acid sample compared to a nucleic acid sample of a non-treated control plant, plant tissue or cell.

5. A nucleic acid molecule obtainable by the method of claim 4, which preferably comprises the nucleotide sequence of SEQ ID NO: 1.

6. The nucleic acid molecule of claim 5, wherein the enzymatic activity and/or substrate or product specificity of the encoded enzyme has been altered.

7. A polypeptide encoded by the nucleic acid molecule of claim 5 or 6.

8. An antibody recognizing specifically the polypeptide of claim 7.

9. A host cell, preferably transgenic plant cell comprising a nucleic acid molecule of claim 5 or 6, or a vector comprising said nucleic acid molecule, wherein the presence of said nucleic acid molecule leads to a change in the content of a depside compared to the wild type plant.

10. A process for the production of depsides, which process comprises
(a) subjecting a substrate comprising phenolic carboxylic acids to a host cell of claim 9 or a culture medium thereof or to a polypeptide of claim 7; and
(b) isolating the depsides so produced.
